# EUROPEAN PATENT APPLICATION

(11) **EP 4 501 954 A1**
(43) Date of publication of application: **05.02.2025**
(21) Application number: 23189184.7
(22) Date of filing: 02.08.2023
(51) Int. Cl.: C07K 14/725, C07K 14/705, C12N 5/0783

(54) **A NUCLEIC ACID ENCODING A CD8 ALPHA BETA CO-RECEPTOR, CELLS AND PHARMACEUTICAL COMPOSITIONS COMPRISING THE SAME**

(71) Applicant: Max-Delbrück-Centrum für Molekulare Medizin in der Helmholtz-Gemeinschaft, 13125 Berlin (DE)
(72) Inventor: Lorenz, Felix, 13129 Berlin (DE); Clauß, Julian, 13086 Berlin (DE); Edes, Inan, 13127 Berlin (DE); Stahn, Rainer, 13125 Berlin (DE); Sada Japp, Alberto, 10439 Berlin (DE)
(74) Representative: Moré, Solveig Helga

(57) **Abstract**

The present invention relates to the field of adoptive immune cell therapy, e.g., adoptive T cell therapy. It provides immune cells, such as be gamma delta T cells, CD4 helper T cells, mucosa-associated invariant cells, innate lymphoid cells, NK cells, macrophages or other types of immune cells or a combination thereof, engineered to express a CD8 co-receptor, and it provides an advantageous configuration of a nucleic acid encoding a CD8 alpha beta co-receptor, wherein the nucleic acid comprises a construct capable of mediating expression of the CD8 alpha and CD8 beta chain under the control of one promotor from a polycistronic mRNA. This improves adoptive immune cell therapy, e.g., it may improve function and persistence of engineered immune cells. The nucleic acid can optionally further encode TCR alpha and beta chain constructs. It can also encode a chimeric antigen receptor. The immune cells may express an alpha beta TCR construct as well as a suitable co-receptor, in particular, an MHC I-restricted alpha beta TCR construct as well as a CD8 co-receptor. Pharmaceutical compositions comprising said cells or nucleic acids are also provided. These are useful for treatment of cancer or infectious diseases.

## Description

The present invention relates to the field of adoptive immune cell therapy, e.g., adoptive T cell therapy. It provides immune cells, such as gamma delta T cells, CD4 helper T cells, mucosa-associated invariant cells, innate lymphoid cells, NK cells, macrophages or other types of immune cells or a combination thereof, engineered to express a CD8 co-receptor, and it provides an advantageous configuration of a nucleic acid encoding a CD8 alpha beta co-receptor, wherein the nucleic acid comprises a construct capable of mediating expression of the CD8 alpha and CD8 beta chain under the control of one promotor from a polycistronic mRNA. This improves adoptive immune cell therapy, e.g., it may improve function and persistence of engineered immune cells. The nucleic acid can optionally further encode TCR alpha and beta chain constructs. It can also encode a chimeric antigen receptor. The immune cells may express an alpha beta TCR construct as well as a suitable co-receptor, in particular, an MHC I-restricted alpha beta TCR construct as well as a CD8 co-receptor. Pharmaceutical compositions comprising said cells or nucleic acids are also provided. These are useful for treatment of cancer or infectious diseases.

Several members of the MAGE family of cancer-testis antigens (CTAs) are highly promising targets for immunotherapy as they offer a safe expression profile and are shared by many patients. The CTA melanoma-associated antigen 4 (MAGE-A4) is expressed at high levels in many different types of solid tumors like gastric cancer, head and neck, lung, breast, ovarian, bladder, and esophageal cancer and can be targeted by T cells (1-4). Adoptive transfer of T cells is currently the most efficacious mode-of-action in oncology. Thus, T cell receptor-engineered T cell (TCR-T cell) therapy for the treatment of MAGE-A4-positive (MAGE-A4⁺) solid tumors presents a promising opportunity for a highly effective cancer treatment with broad applicability.

CTC127, an HLA-A*01:01-restricted TCR against an immunodominant epitope of MAGE-A4 was previously provided by the inventors, and it was shown that CD8⁺ cytotoxic T cells expressing CTC127 delayed tumor outgrowth and provided a significant survival benefit. While CD8⁺ cytotoxic T cells (Tc) are considered the major cytolytic cells in the context of anti-tumor immunity, CD4⁺ helper T cells (Th) have several properties that are advantageous for cancer immunotherapy as they exert helper T cell functions supporting the engraftment and survival of cytotoxic T cells. Further, they are also able to directly kill cancer cells. Indeed, preclinical and clinical reports in literature support that the inclusion of both tumor-specific cytotoxic and helper T cell populations in the product leads to improved clinical responses and survival (5-8).

The co-receptors CD8 or CD4 stabilize the interaction between a T cell and a target cell by binding to invariant portions of MHC (HLA) class I or II, respectively, and are thus required for efficient activation of T cells (termed co-receptor dependence) (9-12).

CTC127 is a CD8-dependent TCR and thus nonfunctional when expressed in CD4⁺ helper T cells, which by nature lack a CD8 co-receptor.

CD8 co-receptor constructs have been described in the past and are in clinical development. A CD8 alpha (CD8a) homodimer is disclosed in WO 2020/049496 A1 (13). A CD8 alpha beta (CD8ab) heterodimer is disclosed by WO 2019/204662 A1 (14). In the context of depletion of autoreactive T cells, CD8a homodimeric or CD8ab heterodimeric constructs comprising alpha and beta chain in covalently linked form have been described (WO 2020/208346 A1 (15)).

In light of the prior art, the inventors addressed the problem of providing advantageous constructs that can improve adoptive cell therapy with immune cells, e.g., T-cells, expressing MHC I-restricted co-receptor-dependent TCR.

This problem is solved by the present invention, in particular, by the subject-matter of the claims.

### CD8 ba co-receptors

The invention provides a nucleic acid encoding a CD8 alpha beta co-receptor, wherein the nucleic acid comprises an operon capable of mediating expression of the CD8 alpha chain and CD8 beta chain under the control of a single promotor from a polycistronic mRNA, wherein the nucleic acid segment encoding the CD8 beta chain is more proximal to the promotor than the nucleic acid segment encoding the CD8 alpha chain.

As shown in the experimental part, co-engineering of T cells, e.g., CTC127 T cells with the novel heterodimeric CD8 co-receptor construct of the invention leads to a gain of function in helper T cells in *in vitro* co-culture assays (peptide titration, tumor cell recognition and killing) and to a more rapid and sustained tumor rejection in an NSG mouse model compared to T cells without the CD8 co-receptor. T cell functions are also improved compared to the prior art CD8 construct. The use of this toolbox technology has the potential to greatly improve the efficacy of TCR-engineered T cells by leveraging the powerful functions of helper T cells.

As explained in more detail below, the inventors also surprisingly found that expression of the nucleic acid of the invention provides other immune cells, e.g., gamma delta T cells expressing an alpha beta TCR, with cytotoxic functions and thus allows for engineering of cell types that are of high interest in allogeneic adoptive T cell therapy.

The nucleic acid of the invention encodes a CD8 alpha chain and a CD8 beta chain. Upon expression, CD8 alpha chain and beta chain are produced and present on the cell surface of cells comprising the nucleic acid of the invention. CD8 alpha chain and CD8 beta chain are not covalently linked in the CD8 alpha beta co-receptor of the invention in protein form, i.e., they are not covalently linked at least in the majority of expressed co-receptors, preferably, in at least 70%, at least 80%, at least 90%, at least 95%, essentially all or all CD8ab coreceptors.

In comparison to prior art heterodimeric CD8 constructs e.g., disclosed in WO 2019/204662 A1 (14), the invention provides a CD8 beta alpha (CD8 ba) heterodimer in which the order of the subunits is inverted in the operon. The inventors were able to show that this change provides a benefit to Th cell function.

The nucleic acid of the invention comprises an operon capable of mediating expression of the CD8 alpha chain and CD8 beta chain under the control of a single promotor from a polycistronic mRNA. In the context of the present invention, an operon is a functioning unit of DNA comprising a cluster of genes under the control of a single promotor. Accordingly, the operon comprises nucleic acid segments comprising the CD8 alpha chain and the CD8 beta chain under the control of a single promotor. The genes are transcribed together into a single polycistronic mRNA molecule. The transcription may or may not be further regulated by a repressor or inducer.

The promotor is operably linked to the first gene of the operon. This may be, e.g., the nucleic acid segment encoding the CD8 beta chain. It may also be another gene, e.g., as described below. It is however not the nucleic acid segment encoding the CD8 alpha chain, as the inventors have found it to be advantageous if, on a polycistronic mRNA transcribed from the operon, the segment encoding the CD8 alpha chain is 3' to the segment encoding the CD8beta chain, i.e., downstream. Without intending to be bound by the theory, this may be due to reduced translation of the downstream CD8 alpha chain compared to the upstream CD8 beta chain.

The promotor is capable of mediating transcription of a polycistronic mRNA. It may be a constitutive or inducible promotor. Preferably it is active in human T cells. It may be active in CD4 and CD8 T-cells, or predominantly or only in CD4T-cells. It can also be only or predominantly active in gamma delta T-cells. Preferably, the promotor is active in immune cells, such as CD8 T cells and CD4 T cells and gamma delta T cells and/or NK cells and/or NKT cells, most preferably in all. For example, the promotor can be human EF1a, PGK, beta actin, IF4A1, GAPDH, GRP78, HSP70, beta kinesin, ubiquitin B, CD45, MP71, or CMV promotor. CD45 is an exemplary promotor specifically active in immune cells. Preferably, the promotor is EF1α.

The operon may also comprise a terminator of transcription, preferably, downstream of the nucleic acid segment encoding the CD8 alpha chain.

CD8 alpha and beta chains are typically from the same species. For example, they may be human, mouse, rat, rabbit, chicken, camel monkey, ape, goat, sheep, pig or cow. Typically, in particular, in the context of treatment of human patients, they are human CD8 alpha and beta chains. They may also be genetically engineered forms of the CD8 co-receptor.

CD8 alpha and beta chain are both members of the immunoglobulin superfamily. They are capable of forming a heterodimer comprising an intracellular region, preferably, a cytoplasmic tail, a transmembrane region and an extracellular immunoglobulin variable (IgV)-like extracellular domain. The extracellular IgV-like domain of CD8 alpha can interact with the alpha3 portion of the Class I MHC molecule. This keeps TCRs that also interact with MHC class I close to CD8 upon binding to a target cell with an MHC I molecule presenting the cognate antigen for the TCR. The cytoplasmic tail of the CD8 co-receptor interacts with the tyrosine kinase Lck (lymphocyte specific protein tyrosine kinase) that can phosphorylate the ITAM motives of the CD3 and zeta-chains of the TCR, which initiates a cascade of phosphorylation eventually leading to activation of transcription factors like NFAT, NFKB and AP-1.

In the context of the invention, the CD8 alpha chain can be any of the isoforms of human CD8 alpha, i.e., 1, 2 (a secreted isoform), or 3, Optionally, as in Example 1 and 2, it is CD8 alpha 1 having SEQ ID NO: 2.

In the context of the invention, the CD8 beta chain can be any of the isoforms of human CD8 beta, i.e., 1, 2, 3 (a secreted isoform), 4, 5, 6 (a secreted isoform), 7 or 8 (a secreted isoform). Optionally, as in Example 1 and 2, it is CD8 beta 5 having SEQ ID NO: 1.

The alpha and beta isoforms can be combined with each other, e.g., the CD8 can be CD8 alpha 1 beta 1, CD8 alpha 1 beta 2, CD8 alpha 1 beta 3, CD8 alpha 1 beta 4, CD8 alpha 1 beta 5, CD8 alpha 1 beta 6, CD8 alpha 1 beta 7, CD8 alpha 1 beta 8, CD8 alpha 2 beta 1, CD8 alpha 2 beta 2, CD8 alpha 2 beta 3, CD8 alpha 2 beta 4, CD8 alpha 2 beta 5, CD8 alpha 2 beta 6, CD8 alpha 2 beta 7, CD8 alpha 2 beta 8, CD8 alpha 3 beta 1, CD8 alpha 3 beta 2, CD8 alpha 3 beta 3, CD8 alpha 3 beta 4, CD8 alpha 3 beta 5, CD8 alpha 3 beta 6, CD8 alpha 3 beta 7, or CD8 alpha 3 beta 8. Preferably, it is CD8 alpha 1 beta 5. To avoid that soluble CD8alpha beta heterodimer is produced, preferably, at least one of the alpha or beta chain preferably is not a secreted isoform lacking a transmembrane domain. CD8 alpha and/or CD8beta transmembrane domains can be transmembrane domains from other proteins, e.g., from CD4, however, they are preferably not fusion proteins. Sequences of wildtype human CD8 alpha and beta chains that may be used are disclosed herein.

Alternatively, modified sequences may be used, e.g., a variant CD8 alpha or CD8 beta chain comprising one or more mutations which increase the binding affinity for the alpha3 portion of MHC class I compared to the wildtype CD8 alpha chain or the CD8 beta chain. For example, high affinity mutants of CD8 alpha generated and characterized using the in vitro evolution method of Wang et al. 2011 (16). Modified sequences or variants preferably have at least 95% amino acid identity with the sequence for a wildtype isoform of CD8 alpha or beta chain e.g., as recited herein, optionally, at least 95% amino acid identity with the sequence of SEQ ID NO: 1 or 2, respectively.

A polycistronic RNA comprises nucleic acid segments encoding at least two proteins expressed from the same promotor. In the context of the invention, a nucleic acid segment is a part of a continuous nucleic acid that comprises other nucleic acid segments. It is typically defined as encoding a specific functionality. For example, a nucleic acid segment may comprise a promotor, a terminator, a functional element such as a cleavage site, or encode a CD8 alpha chain or a CD8 beta chain or a TCR alpha chain construct or a TCR beta chain construct.

In a preferred embodiment, the nucleic acid segment encoding the CD8 beta chain is linked to the nucleic acid segment encoding the CD8 alpha chain via a nucleic acid segment encoding a cleavage site.

The cleavage site may be any sequence which enables two (or more) polypeptides to become separated, e.g., as disclosed in WO 2020208346 A1 (15).

The term cleavage is used herein for convenience, but the cleavage site may cause the peptides to separate into individual entities by a mechanism other than classical cleavage. For example, for the Foot-and-Mouth disease virus (FMDV) 2A self-cleaving peptide (see below), various models have been proposed for to account for the cleavage activity: proteolysis by a host-cell proteinase, autoproteolysis or a translational effect (Donnelly et al. (2001)) (17).). While the exact molecular mechanism of 2A-peptide-mediated cleavage is still unknown, it is believed that it involves ribosomal "skipping" of glycyl-prolyl peptide bond formation rather than true proteolytic cleavage. The exact mechanism of such cleavage is not important for the purposes of the present invention, as long as the cleavage site, when positioned between nucleic acid sequences which encode proteins, causes the proteins to be expressed as separate entities.

The cleavage site preferably is a self-cleaving peptide. This term refers to a peptide which functions such that when the polypeptide comprising the proteins and the self-cleaving peptide is produced, it is immediately cleaved or separated into distinct and discrete first and second polypeptides without the need for any external cleavage activity.

The self-cleaving peptide preferably is a 2A element, e.g., a P2A element, a T2A element, an E2A element or an F2A element. These elements may be derived from an aphtho- or a cardiovirus. The primary 2A/2B cleavage of the aptho- and cardioviruses is mediated by 2A cleaving at its own C-terminus. In apthoviruses, such as foot-and-mouth disease viruses (FMDV) and equine rhinitis A virus, the 2A region is a short section of about 18 amino acids, which, together with the N-terminal residue of protein 2B (a conserved proline residue) represents an autonomous element capable of mediating cleavage at its own C-terminus (Donnelly et al. (2001) as above) (17).

2A elements have been found in picornaviruses other than aptho- or cardioviruses, Picornaviruslike insect viruses, type C rotaviruses and repeated sequences within Trypanosoma spp and a bacterial sequence (Donnelly et al. (2001) as above) (17). 2A elements peptides share a core sequence motif of DXEXNPGP (SEQ ID NO: 7).

Four members of 2A peptides family are frequently used in life science research, and are preferred 2A elements of the invention. They are P2A, E2A, F2A, and T2A. F2A is derived from foot-and-mouth disease virus 18; E2A is derived from equine rhinitis A virus; P2A is derived from porcine teschovirus-1 2A; T2A is derived from thosea asigna virus 2A:

| | |
|---|---|
| T2A | EGRGSLL TCGDVEENPGP (SEQ ID NO: 3) |
| P2A | ATNFSLLKQAGDVEENPGP (SEQ ID NO: 4) |
| E2A | QCTNYALLKLAGDVESNPGP (SEQ ID NO: 5) |
| F2A | VKQTLNFDLLKLAGDVESNPGP (SEQ ID NO: 6) |

Adding the optional linker "GSG" (Gly-Ser-Gly) on the N-terminal of a cleavage site, e.g., a self-cleaving peptide such as a 2A peptide helps with efficiency, and is therefore preferred for all cleavages sites, e.g., P2A elements. There may also be a linker such as a GSG linker C-terminal of cleavage sites.

Linkage thus comprises direct linkage or linkage via a linker.

Different 2A peptides have different efficiencies of self-cleaving, T2A and P2A being the most and F2A the least efficient. Therefore, up to 50% of F2A-linked proteins can remain in the cell as a fusion protein, which might cause some unpredictable outcomes, including a gain of function. One study reported that 2A sites cause the ribosome to fall off approximately 60% of the time, and that, together with ribosome read-through of about 10% for P2A and T2A, this results in reducing expression of the downstream peptide chain by about 70%. However, the level of drop-off detected in this study varied widely depending on the exact construct used, with some constructs showing little evidence of drop-off; furthermore, within a tri-cistronic transcript it reported a higher level of ribosome drop-off after one 2A sequence than after two 2As combined, which is at odds with a linear model of translation.

A P2A element is preferred in the context of the invention, e.g., in combination with a GSG linker directly N-terminal to the P2A element. Accordingly, the nucleic acid of the invention preferably comprises the nucleic acid segment encoding the CD8 beta chain linked to the nucleic acid segment encoding the CD8 alpha chain via a nucleic acid segment encoding a P2A element, wherein, optionally, between the nucleic acid segment encoding the CD8 beta chain and the nucleic acid segment encoding the P2A element, there is a nucleic acid fragment encoding a linker such as a GSG linker directly upstream of the P2A element.

The cleavage site may be self-cleaving, such that when the polypeptide is produced, it is immediately cleaved into individual peptides without the need for any external cleavage activity.

Alternatively, the cleavage site may be a enzymatic cleavage site, such as a furin cleavage site. Furin is an enzyme which belongs to the subtilisin-like proprotein convertase family. The members of this family are proprotein convertases that process latent precursor proteins into their biologically active products. Furin is a calcium-dependent serine endoprotease that can efficiently cleave precursor proteins at their paired basic amino acid processing sites. Examples of furin substrates include proparathyroid hormone, transforming growth factor beta 1 precursor, proalbumin, pro-betasecretase, membrane type- 1 matrix metalloproteinase, beta subunit of pronerve growth factor and von Willebrand factor. Furin cleaves proteins just downstream of a basic amino acid target sequence (canonically, Arg-X-(Arg/Lys)-Arg') and is enriched in the Golgi apparatus.

The cleavage site may also be a Tobacco Etch Virus (TEV) cleavage site. TEV protease is a highly sequence-specific cysteine protease which is chymotrypsin-like proteases. It is very specific for its target cleavage site and is therefore frequently used for the controlled cleavage of fusion proteins both in vitro and in vivo. The consensus TEV cleavage site is ENLYFQS (SEQ ID NO: 8), wherein the cleavage is between Q and S. Mammalian cells, such as human cells, do not express TEV protease. Thus, in embodiments in which the present nucleic acid construct comprises a TEV cleavage site and is expressed in a mammalian cell - exogenous TEV protease must also be expressed in the mammalian cell.

In an alternative embodiment, an internal ribosome entry site (IRES) is located between the nucleic acid segment encoding the CD8 beta chain and the nucleic acid segment encoding the CD8 alpha chain. An IRES is an RNA element that allows for translation initiation in a cap-independent manner, as part of the greater process of protein synthesis. In eukaryotic translation, initiation typically occurs at the 5' end of mRNA molecules, since 5' cap recognition is required for the assembly of the initiation complex. They are described as distinct regions of RNA molecules that are able to recruit the eukaryotic ribosome to the mRNA. This process is also known as cap-independent translation. It has been shown that IRES elements have a distinct secondary or even tertiary structure, but similar structural features at the levels of either primary or secondary structure that are common to all IRES segments have not been reported to date. For example, an IRES disclosed in Hellen et al. (18) is used in the context of the invention. Preferably, the IRES is SEQ ID NO: 9 or SEQ ID NO: 15.

The nucleic acid of the invention may further encode a TCR alpha chain construct and a TCR beta chain construct or a chimeric antigen (CAR) receptor, preferably, a TCR alpha chain construct and a TCR beta chain construct. The TCR alpha chain construct and the TCR beta chain construct together form a TCR construct that is capable of recognizing an epitope such as a cancer epitope in the context of an MHC molecule, i.e., it is MHC-restricted. Preferably, it is MHC class I restricted. TCR alpha chain construct and a TCR beta chain construct can be expressed from different promotors, which can be identical or distinct to the promotor controlling expression of the CD8 coreceptor chains. In the context of the invention, MHC class I is MHC class la, i.e., in the human system, HLA-A, HLA-B or HLA-C. The TCR alpha chain construct and the TCR beta chain construct (or the chains that together encode the CAR) are preferably both expressed from the same promotor, i.e., they form one operon. The operon can be distinct from the operon encoding the CD8 coreceptor. The promotors can be the same or different, E.g., they can be independently selected from the list of promotors recited herein.

Of course, it is also possible that CD8 alpha and beta chains on the one hand and TCR alpha and beta chains on the other hand are encoded on different nucleic acids or in different vectors. They may be transfected at the same time, but that is not required. For example, a pre-existing TCR-transgenic cell may be later endowed with a CD8 co-receptor of the invention.

Alternatively, it is possible that each chain is encoded by a separate operon or a separate nucleic acid. Chains can also be mixes, e.g., with one nucleic acid and, optionally, one operon encoding CD8b and the TCR alpha or beta chain construct and another encoding CD8a and the other of the alpha or beta chain construct, or with one nucleic acid and, optionally, one operon encoding the TCR alpha or beta chain construct and CD8b, and another encoding the other of the alpha or beta chain construct and CD8a (each in that order from the promotor),

In one preferred embodiment, however, the operon comprises nucleic acid segments encoding
- the CD8 alpha chain and the CD8 beta chain and
- a TCR alpha chain construct and a TCR beta chain construct or a chimeric antigen receptor.

Preferably, the operon encodes a TCR alpha chain construct and a TCR beta chain construct. Accordingly, said polypeptides can all be expressed from the same promotor. This can be advantageous to make sure that all cells expressing the TCR also express the CD8 co-receptor and vice versa.

These segments can be in different configurations, as long as the segment encoding the CD8 beta chain is more proximal to the promotor than the segment encoding the CD8 alpha chain. In a preferred embodiment, the operon can comprise the nucleic acid sequence of SEQ ID NO: 10 (nucleic acid segments encoding the CD8 beta chain linked, via a nucleic acid segment encoding a cleavage site, to the nucleic acid segment encoding the CD8 alpha chain).

For example, a) the promotor may be operably linked to the nucleic acid segment encoding the CD8 beta chain, which is linked, via a nucleic acid segment encoding a cleavage site, to the nucleic acid segment encoding the CD8 alpha chain; or
b) the promotor may be operably linked to a nucleic acid segment encoding one of the TCR alpha chain construct or TCR beta chain construct (e.g., the TCR beta chain construct), which is, via a nucleic acid segment encoding a cleavage site, linked to the nucleic acid segment encoding the other of the TCR alpha chain construct or TCR beta chain construct (e.g., the TCR alpha chain construct), which is, via a nucleic acid segment encoding a cleavage site, linked to the nucleic acid segment encoding the CD8 beta chain, which is, via a nucleic acid segment encoding a cleavage site, linked to the nucleic acid segment encoding the CD8 alpha chain; or
c) the promotor may be operably linked to the nucleic acid segment encoding the CD8 beta chain, which is, via a nucleic acid segment encoding a cleavage site, linked to the nucleic acid segment encoding the CD8 alpha chain, which is linked, via a nucleic acid segment encoding a cleavage site, to the nucleic acid segment encoding one of the TCR alpha chain construct or TCR beta chain construct, which is, via a nucleic acid segment encoding a cleavage site, linked to the nucleic acid segment encoding the other of the TCR alpha chain construct or TCR beta chain construct, or
d) the promotor is operably linked to the nucleic acid segment encoding the CD8 beta chain, which is, via a nucleic acid segment encoding a cleavage site, linked to the nucleic acid segment encoding the CD8 alpha chain, which is linked, via a nucleic acid segment encoding a cleavage site, to the nucleic acid segment encoding a selection gene (e.g. DHFR, IMPDH2 or truncated NGFR), which is linked, via a nucleic acid segment encoding a cleavage site, to the nucleic acid segment encoding one of the TCR alpha chain construct or TCR beta chain construct, which is, via a nucleic acid segment encoding a cleavage site, linked to the nucleic acid segment encoding the other of the TCR alpha chain construct or TCR beta chain construct; or
e) the promotor is operably linked to the nucleic acid segment encoding the CD8 beta chain, which is, via a nucleic acid segment encoding a cleavage site, linked to the nucleic acid segment encoding one of the TCR alpha chain construct or TCR beta chain construct, which is, via a nucleic acid segment encoding a cleavage site, linked to the nucleic acid segment encoding the CD8 alpha chain, which is, via a nucleic acid segment encoding a cleavage site, linked to the nucleic acid segment encoding the other of the TCR alpha chain construct or TCR beta chain construct; or
f) the promotor is operably linked to a nucleic acid segment encoding one of the TCR alpha chain construct or TCR beta chain construct, which is, via a nucleic acid segment encoding a cleavage site, linked to the nucleic acid segment encoding the CD8 beta chain, which is, via a nucleic acid segment encoding a cleavage site, linked to the nucleic acid segment encoding the other of the TCR alpha chain construct or TCR beta chain construct, which is, via a nucleic acid segment encoding a cleavage site, linked to the nucleic acid segment encoding the CD8 alpha chain.

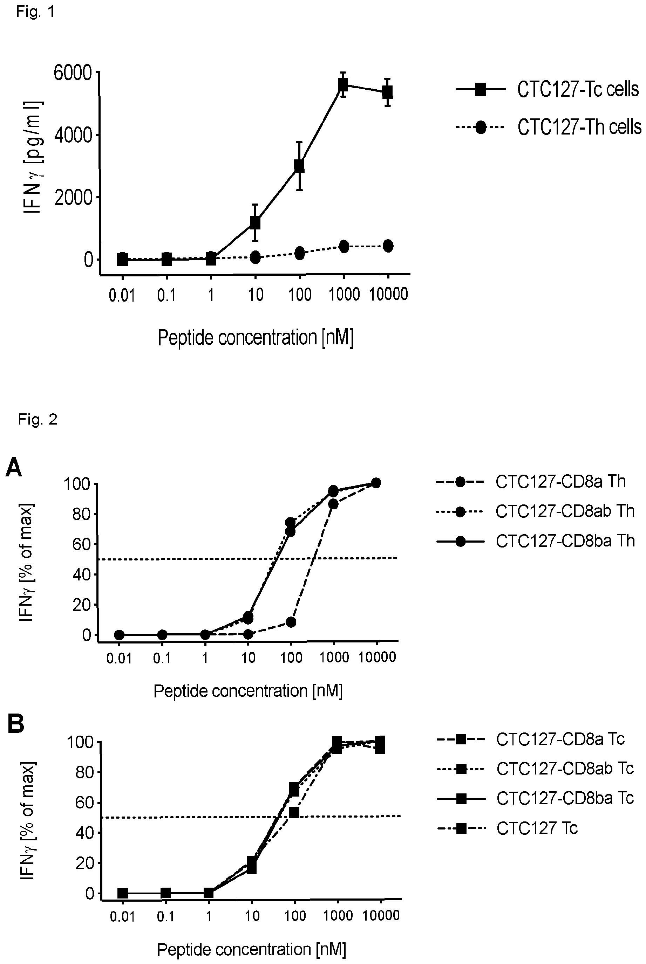

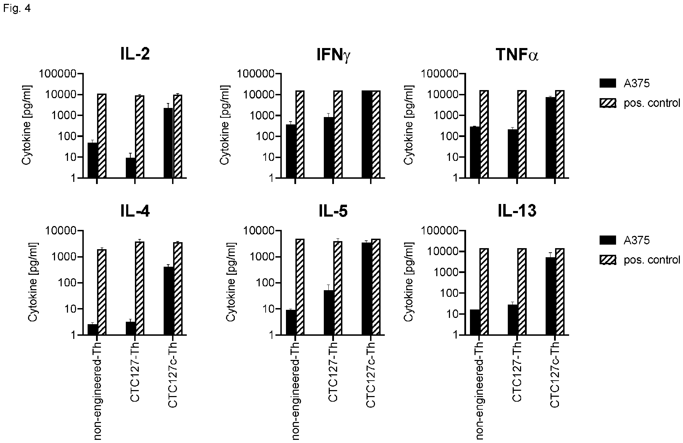

The construct used in example 1 below is a construct of a) (Fig. 1-3) or b) (Fig. 4-7), wherein all cleavage sites are P2A elements.

Other genetic elements, in addition to nucleic acid segments encoding the TCR alpha chain construct and TCR beta chain construct may additionally be comprised in the operon, e.g., encoding another receptor enhancing the function of the immune cells, and/or a selection gene, e.g., DHFR for methotrexate resistance. An additional genetic element may e.g., be located between the promotor and next to the coding element of the operon, e.g., between the promotor and the nucleic acid segment encoding the CD8 beta chain, which is linked, via a nucleic acid segment encoding a cleavage site, to the nucleic acid segment encoding the CD8 alpha chain (as in configuration a), or between the promotor and the nucleic acid segment encoding one of the TCR alpha chain construct or TCR beta chain construct, which is, via a nucleic acid segment encoding a cleavage site, linked to the nucleic acid segment encoding the other of the TCR alpha chain construct or TCR beta chain construct, which is, via a nucleic acid segment encoding a cleavage site, linked to the nucleic acid segment encoding the CD8 beta chain, which is, via a nucleic acid segment encoding a cleavage site, linked to the nucleic acid segment encoding the CD8 alpha chain (as in configuration b), or between the promotor and the nucleic acid segment encoding the CD8 beta chain, which is, via a nucleic acid segment encoding a cleavage site, linked to the nucleic acid segment encoding the CD8 alpha chain, which is linked, via a nucleic acid segment encoding a cleavage site, to the nucleic acid segment encoding one of the TCR alpha chain construct or TCR beta chain construct, which is, via a nucleic acid segment encoding a cleavage site, linked to the nucleic acid segment encoding the other of the TCR alpha chain construct or TCR beta chain construct (as in configuration c).

An additional genetic element may alternatively (or optionally, in addition) be located distal to the genetic element most distal to the promotor enlisted in any of a, b, or c above. It may alternatively (or optionally, in addition) be located between the genetic elements encoding the TCR alpha and beta chain constructs on the one side and the CD8 beta and alpha chains in any of configurations a, b or c above.

Optionally, all cleavage sites are the same, e.g., all cleavage sites may be 2A elements, e.g., P2A elements.

Alternatively, the cleavage sites may also be distinct. Preferably, the nucleic acid segments encoding TCR alpha and beta chains and the nucleic acid segments encoding the CD8 beta and alpha chains may each be linked via a nucleic acid segments encoding a cleavage site such as a 2A element, in particular, a P2A element, and the nucleic acid segments encoding both TCR chains on one side and the nucleic acid segments encoding both CD8 beta and alpha chains on the other side may be linked via a nucleic acid encoding an IRES site.

A TCR alpha and/or beta chain construct may comprise all characteristics or domains corresponding to its native counterpart, i.e., a TCR alpha or beta chain, but this is not essential. Preferably, the TCR alpha and/or beta chain construct comprises at least a variable region, or a variable and a constant region, e.g., the variable and/or constant region having at least 60%, at least 70%, at least 80%, at least 90% or at least 95% sequence identity to a human variable or constant TCR region.

The TCR alpha chain constructs and/or the TCR beta chain constructs preferably comprise a constant region. For adoptive TCR-T cell therapy, it is preferred that the TCR construct comprises full length TCR alpha and beta chains comprising variable and constant regions, including transmembrane regions. The constant region may be a human constant region, a murine constant region or a chimeric constant region such as a minimally murine constant region. The TCR construct may be of essentially or exclusively human origin to minimize immunogenicity. To prevent pairing with endogenous TCR chains, the constructs of the invention may optionally contain one or more, e.g., 1-5, 1-10 or 1-20, preferably, 9 amino acid exchanges in comparison to a human sequence (19).

To prevent pairing with endogenous TCR chains, the constant region of the TCR alpha and beta chain construct may be a murine constant region (20), e.g, it may have at least 80% or at least 90% sequence identity to a murine constant region. TCRs with a murine constant region typically have a stronger expression than with human constant regions.

Additional cysteines may alternatively or additionally be provided to enable the formation of an additional disulfide bond between the TCR chains (21, 22). In addition, codon modification of the TCR sequence may be used to enhance the functional expression of the transgenic TCR (23). As both alpha and beta TCR construct are preferably expressed from one promotor, e.g., linked by a cleavage site such as a 2A element (e.g. P2A) a stoichiometric expression of both chains can be achieved (24). This results in an enhanced functional expression of the transgenic TCR.

The construct may also be a chimeric antigen receptor (CAR), or part of it, wherein, e.g. a human TCR variable region may be linked to a different immunoglobulin constant domain, e.g. an IgG constant domain, and/or to an antibody domain capable of specifically binding to an antigen, preferably, in combination with a CD8 co-receptor, a CAR specific for a peptide-MHC I complex.

Single chain constructs (scTCR) are encompassed as well as heterodimeric TCR constructs. A scTCR can comprise a variable region of a first TCR chain construct (e.g., an alpha chain) and an entire (full-length) second TCR chain (e.g., a beta chain), or vice versa. Furthermore, the scTCR can optionally comprise one or more linkers which join the two or more polypeptides together. The linker can be, for instance, a peptide which joins together two single chains. Also provided is such a scTCR, which is fused to a cytokine, e.g., a human cytokine, such as IL-2, IL-7, IL-12 or IL-15. If the TCR is a single chain construct, only the single chain is encoded by the nucleic acid of the invention or the operon instead of the alpha and beta chain constructs.

To enable specific recognition of the epitope in the context of the MHC, in particular, for therapeutic purposes in human patients, the nucleic acid of the invention encodes one TCR alpha and one beta chain construct of a TCR capable of recognizing an epitope in the context of a human MHC, preferably, in the context of human MHC I.

In a preferred embodiment, the TCR construct is capable of recognizing an epitope from a tumor antigen in complex with a human MHC I, e.g., an antigen from a solid tumor, e.g., a MAGE antigen such as MAGE-A4.

Typically, the nucleic acid sequences provided by the invention are codon-optimized for expression in human cells.

In one embodiment, the nucleic acid of the invention comprises SEQ ID NO: 11 or a sequence having at least 80%, e.g., at least 90%, at least 95% or at least 99% sequence identity thereto. In a preferred embodiment, the nucleic acid of the invention comprises SEQ ID NO: 14 or a sequence having at least 80%, e.g., at least 90%, at least 95% or at least 99% sequence identity thereto. In SEQ ID NO: 11, the TCR chains are more proximal to the promotor than the CD8 chains, and in SEQ ID NO: 14, they are more distal. Interestingly, expression from SEQ ID NO: 14 is better than from SEQ ID NO: 11 (data not shown). The nucleic acid segments encoding the alpha and/or beta chain constructs of the nucleic acid can be substituted for a different TCR alpha and beta chain construct-encoding sequence. Accordingly, the nucleic acid of the invention may have at least 80%, e.g., at least 90%, at least 95% or at least 99% sequence identity to base pairs 1870 - 3369 of SEQ ID NO: 11 or, preferably, base pairs 1 - 1500 of SEQ ID NO: 14.

The nucleic acid of the invention may be a vector, such as an expression vector. It typically is DNA. The nucleic acid may e.g. be a minicircle, a plasmid, doggybone DNA or RNA.

Recently DNA constructs using doggybone DNA (dbDNA), synthetic closed linear DNA, have been developed. The cell-free process relies on the use of amplification, e.g., with Phi29 DNA polymerase, wherein amplification of the template is followed by incubation with protelomerase TelN to complete individual closed linear DNA. The resulting dbDNA may e.g., contain the required sequences (e.g., encoding CD8 beta chain lined by a cleavage site to CD8 alpha chain, and optionally also TCR alpha and beta chain constructs), a promoter and a poly A tail, but lacks bacterial sequences such as an antibiotic resistance gene.

A minicircle is derivable from a plasmid wherein bacterial sequences such as the origin of replication have been excised. Minicircles are small (typically smaller than 5 kb, preferably, about 4kb) circular plasmid derivatives that have been freed from all prokaryotic vector parts. They have been applied as transgene carriers for the genetic modification of mammalian cells, with the advantage that, since they contain no bacterial DNA sequences, they are less likely to be perceived as foreign and destroyed. The smaller size of minicircles also extends their cloning capacity and facilitates their delivery into cells, e.g., using transposases. Suitable nucleic acids and protocols are e.g., disclosed in WO 2017/158019 A1 (25).

Minicircles lack an origin of replication, so they typically do not replicate within the target cells and the encoded genes will disappear as the cell divides. A novel addition to the field are nonviral selfreplicating minicircles, which owe this property to the presence of a S/MAR-Element.

Plasmids (circular DNA comprising an origin of replication can also be used in the context of the invention, but it is preferred that less DNA needs to be transferred, so preferably, the nucleic acid is a doggybone DNA or a minicircle, most preferably, a minicircle.

In one embodiment, the DNA is a transposon or comprises a transposon. Consequently, the operon may be flanked by inverted repeats capable of being mobilized by a transposase, preferably, by a Sleeping Beauty transposase such as SB100X. Suitable transposases are e.g., disclosed in WO 2009/003671 A2 (26). Suitable transposons are e.g., disclosed in WO 2017/158029 A1 (27). For example, the vector may be p2 vector, or, preferably, a p4 or p5 vector.

The vector may also be a viral vector, e.g., a lentiviral vector, a gamma retroviral vector, an adenoviral vector or an AAV. The vector may also be a vector suitable for CRISPR/Cas homology directed repair (HDR) genome engineering combined with a guide RNA in a kit. The guide RNA may, e.g., be homologous to a section of the endogenous TCR alpha, beta, delta or gamma locus. Thus, advantageously, in particular, for alpha beta T cells, the endogenous TCR is rendered nonfunctional by insertion of the nucleic acid of the invention, preventing mispairing and/or GVHD. In gamma delta T cells, expression of the endogenous TCR, which can often have an anti-tumor effect, can also be desired, so other loci can be selected for engineering.

The invention also discloses a method of preparing a cell comprising the nucleic acid of the invention. The vector, e.g., the purified minicircle can be transferred into the recipient cell by transfection, lipofection, jet injection or, preferably, electroporation.

The present invention also provides a cell comprising the nucleic acid of the invention, wherein the cell preferably also expresses the CD8 alpha beta co-receptor. The cell can be a bacterial cell, e.g., *E. coli,* but it is preferably an eukaryotic cell, e.g., a mammalian cell. Typically, for research purposes, the cell is a mouse or human cell. Therapeutic cells are usually human cells, and a human subject is to be treated.

For treatment, alpha beta T cells engineered with the nucleic acid of the invention can be allogeneic, but they are preferably syngeneic, typically, autologous. This avoids immune rejection or the need for immune suppression to avoid Graft versus host disease (GvHD). Alpha beta T cells expressing the CD8 co-receptor of the invention are preferably CD4+ T cells, as CD8+ T cells already naturally express the co-receptor. A mixture of cells comprising CD4+ and CD8+ cells can however also be transfected with the co-receptor of the invention.

The cell of the present invention preferably further expresses a class-I-restricted T cell receptor construct.

It preferably is an immune cell, such as a T cell, NK cell or NKT cell. T cells can be alpha beta T cells or gamma delta T cells, in particular CD4 alpha beta T cells, or more preferably, a mixture of CD4 and CD8 alpha beta T cells and gamma delta T cells.

However, it is one of the advantages of the invention that other immune cells, e.g., gamma delta T cells, NK cells or NKT cells, monocytes or macrophages or B-cells, can also be endowed with cytotoxic functionality against target cells, in particular, against tumor cells, using a nucleic acid of the invention that encodes both CD8 alpha and beta chains and TCR alpha and beta chain constructs. TCR and CD8 can also be encoded on separate nucleic acids. The immune cells expressing both TCR and CD8 can be activated in an advantageous manner by target cells expressing the cognate antigen and presenting it in an MHC-I restricted manner.

In one embodiment, if the cell is a CD4 T cell, it is derived from PBMC (Peripheral blood mononuclear cells). They can be derived from a subject, e.g., from PBMC of the subject, in high amounts, in particular, through stimulation of the T cells. The CD4 T cells can be isolated from other T cells before stimulation, but this is not required. Indeed, it is advantageous if CD4 and CD8 T cells are both engineered with the nucleic acid of the invention, and, thus, not separated from each other. T cells can be isolated from other cells in PBMC, e.g., the concentration of B-cells can be reduced. Suitable methods are e.g., known in the art.

T cells can be stimulated and expanded, e.g., as known in the art, for example, with anti-CD3 or anti-TCR (gamma and/or delta chain) antibodies, optionally, bound to a solid support, preferably, in combination with anti-CD28 antibodies. They can also be stimulated by contact with target cells expressing their cognate antigen (after engineering the cells to express the transgenic TCR) and/or by addition of cytokines, such as IL-1β, IL-2, IL-4, IL-5, IL-7, IL-15, IL-21, IFNγ and/or by addition of bisphosphonates, such as, zoledronate, risedronate, ibandronate, alendronate, olpadronate, neridronate, pamidronate, tiludronate, clodronate, and etidronate.

In a preferred embodiment, the invention provides a composition of cells comprising gamma delta T cells of the invention. The compositions may be purified, or optionally further comprise CD8+ and CD4+ T cells and/or NK cells and/or NKT cells. Advantageously, gamma delta T cells (and, optionally, also the other recited cells) comprise the nucleic acid of the invention and express the CD8 alpha beta co-receptor. In one embodiment, the compositions comprising cells of the invention are obtainable by transfecting PBMC of a subject (e.g., a subject that is to be treated, or an unrelated donor) or cells derived therefrom with the nucleic acid of the invention.

### Immune cells, in particular, gamma delta T cells

Gamma delta T cells are very interesting sub-group of T cells that do not express an alpha beta TCR, but a gamma delta TCR. Ligands for gamma delta TCRs remain largely unknown. Based on the data on gamma delta TCR specificities identified so far, it is generally considered that the receptors bind to their targets in a way independent from MHC-presentation of peptides. They may bind to conformational epitopes, i.e. specific surface structures of proteins and possibly other molecules. For example, some gamma delta TCRs are known to bind to phosphoantigens and aminobisphosphonates that may be induced by metabolic changes in cells such as tumor cells. The target of a Vγ9Vδ2 TCR is known to be a joint spatial and conformational change of CD277 at the cell membrane of cancer cells.

Gamma delta T cells thus have a naturally high tumor cell killing potential. It has also been found that they can naturally infiltrate solid tumors, and that gamma delta T cell-rich tumors have a significantly better prognosis.

Further, when gamma delta T cells are engineered to express alpha beta TCR, in contrast to engineered alpha beta T cells, there is no pairing of exogenous and endogenous alpha and beta TCR chains. Gamma and delta TCR chains do not pair with alpha beta TCR chains. Thus, the risk of autoimmunity caused by such pairing is eliminated (US 2003219463 A1 (33), van der Veeken et al, 2006 (34), van der Veeken et al, 2009 (35)).

Another feature that makes gamma delta T cells particularly interesting for adoptive T cell therapy is that allogeneic gamma delta T cells do not induce GvHD. Since they do not carry classical endogenous alpha beta TCR that recognize epitopes in the context of MHC, they do not recognize "foreign" MHC.

Using allogeneic T cells for adoptive T cell therapy is of high interest, because they can be used as off-the-shelf therapeutics instead of necessitating a cost- and time-intense engineering of the patients' own T cells. Because of scalability, the cost per dose would be about 10 times lower. Further, progressing patients may be immediately treated, and there is a good fitness of the T cells - often in contrast to patients' T cells, which may already be affected by the disease of the patient and/or previous treatments. Manufacturing failures for specific patients can also be avoided, and the cells can be more easily standardized.

Because they do not induce GvHD, gamma delta cells would be ideal for use in allogeneic adoptive T cell therapy. This feature is shared by other immune cells, such as NK cells, but gamma delta cells are additionally more long-lived and can be generated in high cell numbers, e.g., from healthy donors' PBMC.

Allogeneic CAR gamma delta T cells have already been successfully used in a clinical trial targeting CD20 lymphoma (28). They were found to have a favorable safety profile and a 75% overall response rate across all dose levels, as well as 69% complete responses. Circulating CAR gamma delta T cells were detectable through day 28 in blood at the highest dose level. However, it is known in the art that CAR T cells may lead to problems with resistance of the tumor, as the tumor cells can down regulate surface expression of the antigen on which recognition by the CAR is dependent, and such cells are selected for by the therapy.

In light of this, the inventors solved the problem of providing advantageous cells useful for allogeneic gamma delta T cell therapy in the present invention. In particular, the present invention provides, in one embodiment, an immune cell expressing
a) a TCR alpha chain construct and a TCR beta chain construct of a MHC class I restricted TCR construct and
b) a CD8 co-receptor comprising both CD8 alpha and CD8 beta chain,
wherein the immune cell is selected from the group consisting of a gamma delta T cell, an NK cell, an NKT cell, a mucosa-associated invariant T (MAIT) cell, an innate lymphoid cell (ILC), a CD4+ T helper cell, a monocyte, a macrophage and a B cell.

The co-receptor is CD8 comprising both CD8 alpha and CD8 beta chain, i.e., it is not CD8a. As shown herein, CD8 with alpha and beta chain provides better co-stimulation that CD8a. Preferably, if the immune cell expresses CD8, the nucleic acid encoding the same is the nucleic acid of the invention as described herein.

However, the nucleic acid may also encode a CD8 alpha beta construct i.e., it may be a nucleic acid encoding a CD8 alpha beta co-receptor, wherein the nucleic acid comprises an operon capable of mediating expression of the CD8 alpha chain and CD8 beta chain under the control of a single promotor from a polycistronic mRNA, wherein the nucleic acid segment encoding the CD8 alpha chain is more proximal to the promotor than the nucleic acid segment encoding the CD8 beta chain.

The CD8 alpha chain can be any of the isoforms of human CD8 alpha, i.e., 1, 2 (a secreted isoform), or 3, Optionally, as in Example 1 and 2, it is CD8 alpha 1 having SEQ ID NO: 2.

In the context of the invention, the CD8 beta chain can be any of the isoforms of human CD8 beta, i.e., 1, 2, 3 (a secreted isoform), 4, 5, 6 (a secreted isoform), 7 or 8 (a secreted isoform). Optionally, as in Example 1 and 2, it is CD8 beta 5 having SEQ ID NO: 1.

The alpha and beta isoforms can be combined with each other, e.g., the CD8 can be CD8 alpha 1 beta 1, CD8 alpha 1 beta 2, CD8 alpha 1 beta 3, CD8 alpha 1 beta 4, CD8 alpha 1 beta 5, CD8 alpha 1 beta 6, CD8 alpha 1 beta 7, CD8 alpha 1 beta 8, CD8 alpha 2 beta 1, CD8 alpha 2 beta 2, CD8 alpha 2 beta 3, CD8 alpha 2 beta 4, CD8 alpha 2 beta 5, CD8 alpha 2 beta 6, CD8 alpha 2 beta 7, CD8 alpha 2 beta 8, CD8 alpha 3 beta 1, CD8 alpha 3 beta 2, CD8 alpha 3 beta 3, CD8 alpha 3 beta 4, CD8 alpha 3 beta 5, CD8 alpha 3 beta 6, CD8 alpha 3 beta 7, or CD8 alpha 3 beta 8. Preferably, it is CD8 alpha 1 beta 5. To avoid that soluble CD8alpha beta heterodimer is produced, preferably, at least one of the alpha or beta chain preferably is not a secreted isoform lacking a transmembrane domain. CD8 alpha and/or CD8beta transmembrane domains can be transmembrane domains from other proteins, e.g., from CD4, however, they are preferably not fusion proteins. Sequences of wildtype human CD8 alpha and beta chains that may be used are disclosed herein.

Alternatively, modified sequences may be used, e.g., a variant CD8 alpha or CD8 beta chain comprising one or more mutations which increase the binding affinity for the alpha3 portion of MHC class I compared to the wildtype CD8 alpha chain or the CD8 beta chain. For example, high affinity mutants of CD8 alpha generated and characterized using the in vitro evolution method of Wang et al. 2011 (16). Modified sequences or variants preferably have at least 95% amino acid identity with the sequence for a wildtype isoform of CD8 alpha or beta chain e.g., as recited herein, optionally, at least 95% amino acid identity with the sequence of SEQ ID NO: 1 or 2, respectively.

The TCR construct is MHC class I restricted, i.e., if it is capable of recognizing an epitope in the context of MHC class I. The co-receptor CD8 can interact with said complex.

The immune cell may be, e.g., an immune cell that does not naturally express an alpha beta T cell receptor, preferably, a gamma delta T cell.

Gamma delta T cells can be, e.g., derived from PBMC or T cells isolated therefrom. They can be enriched by antibodies against the gamma and/or delta TCR chains, and/or by depletion of alpha beta T cells and/or by stimulation with bisphosphonates and/or by stimulation with anti-CD3 antibodies (29).

The inventors could show that a 100fold expansion of TCR-engineered gamma delta T cells, e.g., from PBMC of a healthy donor, was possible. Protocols for gamma delta T cell expansion that may be used are known in the art, e.g., Tan et al. (29), US 2003219463 A1 (33), van der Veeken et al, 2006 (34), van der Veeken et al, 2009 (35) or WO2019/104269 A1 (36). The gamma delta T cells of the invention, which carry an alpha beta TCR construct, can be stimulated with anti-CD3 antibodies which stimulates all subsets of gamma delta T cells. Alternatively, the Vγ9Vδ2 subset can be specifically stimulated using isopentenyl pyrophosphate (IPP), bromohydrin pyrophosphate (BrHPP) or bisphosphonates (e.g. zoledronate). Gamma delta T cells can also be generated from iPSC, e.g., human iPSC. Alternatively, engineered gamma delta T cells expressing the co-receptor and an MHC class I restricted TCR can be expanded antigen-specifically with cells expressing the target antigen, and/or cells coated with the cognate peptide.

The inventors have also shown that co-expression of a CD8 co-receptor, in particular, of a CD8 alpha beta co-receptor and a MHC class I restricted TCR construct endows gamma delta T cells with the functions typically seen in CD8 cytotoxic T cells, e.g., IFN-gamma production upon co-culture with tumor cells expressing the relevant epitope and MHC I, as well as cytotoxic functions. In contrast, gamma delta T cells expressing only the TCR without the CD8 co-receptor fail to elicit the full breadth of responses of the gamma delta T cells against the targeted solid tumor cells. Accordingly, the gamma delta T cells of the invention co-expressing CD8 co-receptor and a MHC class I restricted TCR construct can advantageously be used in adoptive T cell therapy, e.g., in allogeneic adoptive T cell therapy.

In contrast to CAR T cells, T-cells expressing MHC class I restricted TCR can target intracellular antigens, such as tumor antigens. This also applies for intracellular solid tumor antigens, such as MAGE, e.g., MAGE-A4. Downregulation of cell surface expression is thus not sufficient for evading the action of such T cells.

The inventors have additionally shown that gamma delta T cells can naturally sense HLA (Human leukocyte antigen / MHC class I) loss by tumor cells. For example, the HLA-negative cell line K562 is recognized by non-engineered gamma delta T cells, probably though innate immune function. This may provide an additional mode of action if tumor cells try to escape through downregulation of HLA.

Gamma delta T cells may also be derived from the patient who is to be treated with the cells after engineering. However, the donor from which gamma delta T cells may be derived preferably is a healthy donor. Accordingly, the donor is not diagnosed with a cancer, and, typically, also not with an infective disease and/or other disease. The donor may be a relative, e.g., a sibling of the patient, but, due to lack of GvHD, this is not required. Preferably, the donor shares the MHC I to which the TCR with which the gamma delta T cell is engineered is restricted, and, most preferably, the donor is haploidentical for this MHC. For example, the donor can have two alleles of HLA-A*01 :01, which is the second most frequent HLA allele in Europe. It is also the HLA to which CTC127, a TCR recognizing a MAGA-A4 epitope (WO 2022/243514 A1 (30)), is restricted.

This, together with the fact that gamma delta T cells do not express high levels of MHC II molecules, already largely reduces the risk of host-versus graft reactions. Optionally, other HLA-molecules can be downregulated, e.g., by inhibitory RNA or knocked out by methods such as CRISPR/Cas engineering. Donors can be selected that are also haploidentical for one or, preferably, both other HLA molecules. Further, libraries of gamma delta T cells having the most common HLA-types, preferably, in haploidentical form can be provided, so that suitable gamma delta T cells for a particular patient can be selected.

The immune cell can be a CD4+ T helper cell. Thus, the invention provides a CD4+ CD8 alpha beta + T helper cell that further expresses a TCR construct that is MHC class I restricted. The TCR construct preferably is a heterologous TCR construct, i.e., alpha and beta chains are preferably expressed under the control of a heterologous promotor. The inventors have shown that co-expression of an MHC class I restricted TCR construct and CD8 comprising an alpha and beta chain provides functional T cells that can respond to the target antigen of the TCR (presented by a suitable MHC). The response can comprise killing of target cells and/or secretion of cytokines.

The immune cell can be an innate lymphoid cell (ILC). ILCs are derived from common lymphoid progenitors (CLPs). In response to pathogenic tissue damage, ILCs contribute to immunity via the secretion of signaling molecules, and the regulation of both innate and adaptive immune cells. ILCs are primarily tissue resident cells, found in both lymphoid (immune associated), and non- lymphoid tissues, and rarely in the blood. They are particularly abundant at mucosal surfaces, playing a key role in mucosal immunity and homeostasis. Characteristics allowing their differentiation from other immune cells include the regular lymphoid morphology, absence of rearranged antigen receptors found on T cells and B cells (due to the lack of the RAG gene), and phenotypic markers usually present on myeloid or dendritic cells. Based on the difference in developmental pathways, phenotype, and signaling molecules produced, ILCs can be divided into five groups: NK cells, ILC1s, ILC2s, ILC3s, and lymphoid tissue inducer (LTi) cells (Wikipedia). ILCs do not naturally express a CD4 or CD8 co-receptor, but in the context of the invention, they can be engineered to express a CD8 co-receptor as defined herein. ILCs of the invention in a preferred embodiment express the CD8 ba co-receptor of the invention as well as a MHCI-restricted TCR. Preferred ILC are NK cells.

The immune cell can be an NK (Natural Killer) cell, NKT cell or monocyte, macrophage or B-cell expressing a TCR alpha chain construct and a TCR beta chain construct of a MHCI restricted TCR.

If the immune cell does not naturally express CD3, such as macrophages, B-cells or NK cells, expression of the TCR and the CD8 co-receptor allows for targeting of the immune cell to an environment in which the antigen to which the TCR is directed is expressed. Thus, for example, if the TCR targets an epitope form a tumor antigen, the immune cells can be targeted to the tumor.

Further, optionally, the immune cells may be further engineered to express CD3, thus also allowing for activation of the cells by TCR binding, which can lead to cytotoxic killing of the target cell expressing the cognate antigen, to secretion of cytokines and/or phagocytosis (in particular, in the case of macrophages). If the immune cell is a B cell, the activation may also lead to secretion of antibodies. This may be helpful, e.g., if the B cell further expresses an antibody, e.g., through genetic engineering, that may target a B cell epitope of an antigen such as a tumor antigen. The same or an associated antigen may be targeted by the TCR. In one embodiment, signaling domains of CD3 may be expressed as a fusion protein with the TCR alpha and/or beta chains, e.g., as disclosed in Sebestyén et al. (*31*) and Govers et al. (*32*) .

The immune cell of the invention comprises a nucleic acid encoding the co-receptor and a nucleic acid encoding the TCR alpha chain and TCR beta chain constructs, or, preferably, one nucleic acid encodes both co-receptor and TCR. The nucleic acid segments encoding the chain(s) of the coreceptor and the TCR are operably linked to a heterologous promotor, e.g., a promotor as disclosed herein.

The CD8 co-receptor expressed by the immune cell, e.g., the gamma delta T cells of the invention is a heterodimeric CD8 alpha beta co-receptor, as this leads to significantly higher levels of co-stimulation than expression of a CD8 alpha homodimeric co-receptor. For example, it can be a CD8 ab co-receptor, as disclosed by WO 2019/204662 A1 (14). A CD8ab heterodimeric constructs comprising alpha and beta chain in covalently linked form as described in WO 2020/208346 A1 (15) may also be used.

However, due to the advantageous effects shown by the inventors, the CD8 co-receptor preferably is a CD8 ba co-receptor, i.e., the gamma delta T cell preferably comprises and expresses the nucleic acid of the invention, as described herein.

### Pharmaceutical compositions

The invention also provides a pharmaceutical composition comprising the nucleic acid of the invention or the cell of the invention. The pharmaceutical composition optionally further comprises a suitable buffer and/or excipient.

The pharmaceutical compositions of the invention typically are for intravenous administration. They may comprise pharmaceutically acceptable carriers such as buffers, e.g., physiological saline or PBS. They may comprise excipients, e.g., stabilizers such as SPGA, carbohydrates (e.g. sorbitol, mannitol, starch, sucrose, glucose, dextran), proteins such as albumin or casein, or protein containing agents such as bovine serum or skimmed milk. If the composition does not comprise cells but nucleic acids, it can be a dry composition, e.g., a lyophilized composition. Such compositions typically comprise bulking agents, e.g., non-reducing sugars such as trehalose. They may also comprise buffering agents, e.g., as recited herein.

T cells or other immune cells are typically administered to a subject or patient in a concentration of 1 x 10⁵ - 5 x 10⁹ cells per kg bodyweight. About 1 x 10⁶ - 5 x 10¹¹ cells, e.g., 1 x 10⁸ - 2 x 10¹⁰ cells may be administered to a human subject as a single dose, i.v.. These parameters may be adapted by the medical practitioner, depending, e.g., on the patients age, sex, body weight and medical condition.

The pharmaceutical composition of the invention in one embodiment comprises autologous T cells of the subject which were *in vitro*-engineered to express a nucleic acid of the present invention, e.g., autologous CD4 and CD8 T cells. Alternatively, the subject may also be administered a nucleic acid of the invention, in particularly, an expression vector, for *in vivo* transduction of T cells.

In another embodiment, the pharmaceutical composition comprises the immune cells, e.g., gamma delta T cells of the invention co-expressing, a) a TCR alpha chain construct and a TCR beta chain construct of a MHC class I restricted TCR construct and b) a CD8 co-receptor. Preferably, these cells comprise and express a nucleic acid of the invention. Alternatively, they can also co-express, a) a TCR alpha chain construct and a TCR beta chain construct of a MHC class II restricted TCR construct and b) a CD4 co-receptor.

The present invention also provides the pharmaceutical composition of the invention for use in treatment of a subject in need thereof, in particular, a subject having a cancer or an infectious disease, preferably, a cancer such as a solid cancer. A subject that has a disease, e.g., a cancer or an infectious disease is also designated a patient herein. Preferably, the pharmaceutical composition is for use in in adoptive cell therapy, e.g., adoptive T cell therapy of cancer.

It may be adoptive cell therapy, e.g., adoptive T cell therapy, or T cell receptor (TCR) gene therapy directed to an epitope derived from the cancer-testis antigen MAGEA4 presented on HLA-A*01, e.g., with the TCR CTC127 (as disclosed in WO 2022/243514 A1 (30)) or a variant thereof having at least 90 % sequence identity in the CDR1, CDR2 and CDR3 regions, and preferably, having at least 98% or at least 99% sequence identity to CTC127 over the complete sequence of the variable regions.

In one embodiment, the cells employed for adoptive T cell therapy are allogeneic gamma delta T cells of the invention, preferably expressing a) a TCR alpha chain construct and a TCR beta chain construct of a MHC class I restricted TCR construct and b) a CD8 co-receptor, wherein, optionally, the cells comprise and express the CD8 ba nucleic acid of the invention.

While of course, other antigens or epitopes can also be targeted, optionally, the TCR construct targets an antigen expressed by a solid tumor, e.g., a MAGE antigen such as MAGEA4. The TCR construct can e.g., be CTC127 or a variant thereof as described herein. Said TCR can be expressed in CD4 and CD8 T cells or in gamma delta T cells as described herein.

Also disclosed is a method of treating a subject in need thereof, e.g., a subject having a cancer or an infectious disease, preferably, a cancer such as a solid cancer or a lymphoma. The therapy typically comprises intravenous administration of the pharmaceutical compositions of the invention, preferably, infusion. Typically, before administration of T cells, the patients are pre-conditioned, e.g., by administration of fludarabine and cyclophosphamide (e.g., infusions of 30 mg/m² fludarabine and 500 mg/m² cyclophosphamide), for example, on days 5, 4 and 3 prior to administration of T cells.

The subject typically is a mammalian subject, e.g., a mouse or a human. Preferably, it is a human patient.

An infectious disease may be, e.g., a bacterial infection with multi-resistant bacteria, a viral infection or a fungal infection. Viral infections are well suitable for treatment with adoptive cell therapy with cells, e.g., T cells, having a MHC I-restricted TCR. A viral disease may be a coronavirus-mediated disease, e.g., by SARS- CoV-1, MERS or SARS-CoV-2. It may also be infection with an influenza virus.

The solid cancer can be, e.g., melanoma, gastric cancer, head and neck, lung, breast, ovarian, bladder, and oesophageal cancer. The cancer-testis antigen melanoma-associated antigen 4 (MAGEA4) is expressed at high levels in all of these solid tumors. The cancer can also be lymphoma. For example, adoptive T cell therapy of B cell lymphoma targeting CD19 has been shown to have a high potential. Combination with the CD8 beta alpha co-receptor construct of the invention can further improve such therapies.

The present invention is further illustrated in the following examples with reference to the accompanying figures and sequences, nevertheless, without being limited thereto. For the purposes of the present invention, all references as cited herein are incorporated by reference in their entirety.

### Detailed description of the figures

Fig. 1. CTC127 is CD8-dependent. Tc and Th cells were separately transduced with CTC127 and placed in a co-culture with peptide-loaded K562-A0101. After overnight culture, IFNywas measured in the supernatant by ELISA. Experiment performed in replicates with two T cell donors. Data were pooled. Shown is mean ± SEM. Tc, CD8⁺ cytotoxic T cells; Th, CD4⁺ helper T cells.
Fig. 2. CD8 co-receptor confers functionality to CTC127 Th cells. Tc and Th cells were separately transduced with CTC127 and one of three CD8 co-receptor constructs (a, ab or ba). They were then placed in a co-culture with peptide-loaded K562-A0101. T cell numbers were adjusted based on the individual transduction rates in order to have the same number of TCR-expressing cells in every group. After overnight culture, IFNy was measured in the supernatant by ELISA. IFNy concentration was normalized to percentage of maximum for plotting of the dose-response curves. Experiment performed in replicates with two T cell donors. Data of both donors were pooled. Shown is mean ± SEM. A) Dose-response curves for Th cells expressing CTC127 and the different coreceptor constructs. B) Dose-response curves for Tc cells expressing CTC127 and the different coreceptor constructs.

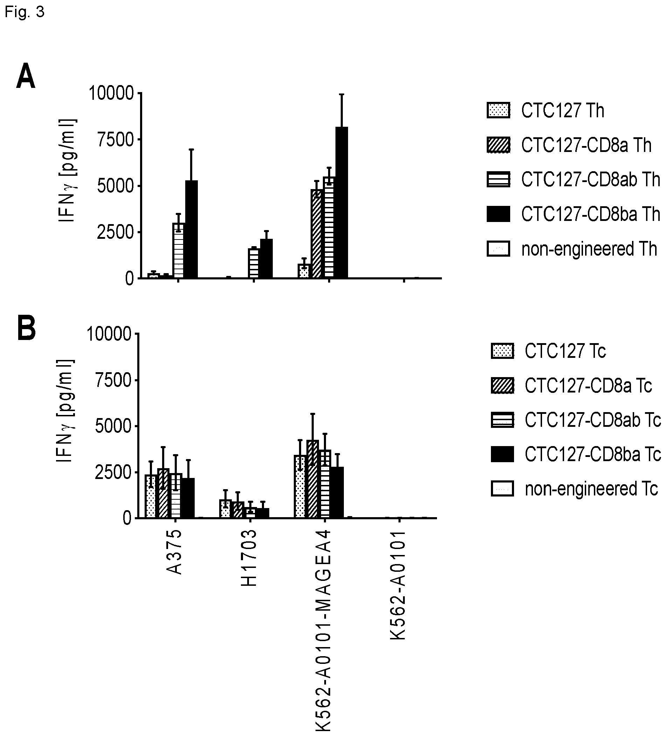

Fig. 3. Th cells expressing CTC127 and a heterodimeric CD8ba co-receptor recognize cancer cells. Tc and Th cells were separately transduced with CTC127 along with one of three CD8 co-receptor constructs (a, ab or ba). They were placed in a co-culture with the MAGE-A4-expressing cancer cell lines A375 and H1703. Engineered K562-A0101-MAGE-A4 and K562-A0101 were used as positive and negative controls, respectively. T cell numbers were adjusted based on the individual transduction rates in order to have the same number of TCR-expressing cells in every group. After overnight culture, IFNy was measured in the supernatant by ELISA. A) Co-culture with Th cells. B) Co-culture with Tc cells. Experiment performed in replicates with two T cell donors. Data were pooled. Shown is mean ± SEM.
Fig. 4. CTC127c-Th cells secrete multiple cytokines that support Tc cells and that also have cytotoxic function. Th cells were transduced with CTC127 or CTC127c and then placed in a co-culture with A375 cancer cells or activated with beads coated with anti-CD3/anti-CD28 (positive control). After 24 hours, cytokine concentration in the supernatant was measured by a multiplex cytometric bead array (CBA). Experiment performed with replicates of two T cell donors. Data were pooled. Shown is mean ± SEM.

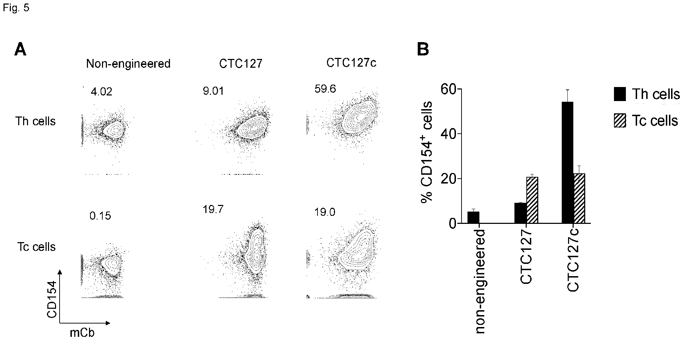

Fig. 5. CTC127c-Th cells express the helper molecule CD154. Tc and Th cells were separately transduced with CTC127 or CTC127c and then placed in a co-culture with A375 cancer cells. 10 µg/ml anti-CD40 antibody was added to the culture to block the CD154-CD40 interaction and subsequent downregulation of CD154. After 24h, CD154 was detected on the surface of the T cells by flow cytometry. Experiment performed in replicates with two T cell donors. A) Flow cytometry plots showing the frequency of CD154⁺ T cells in one donor. Mouse constant beta chain (mCb) staining the TCR CTC127 is shown on the x-axis. B) Summary of CD154 frequency. Mean ± SEM of two T cell donors.

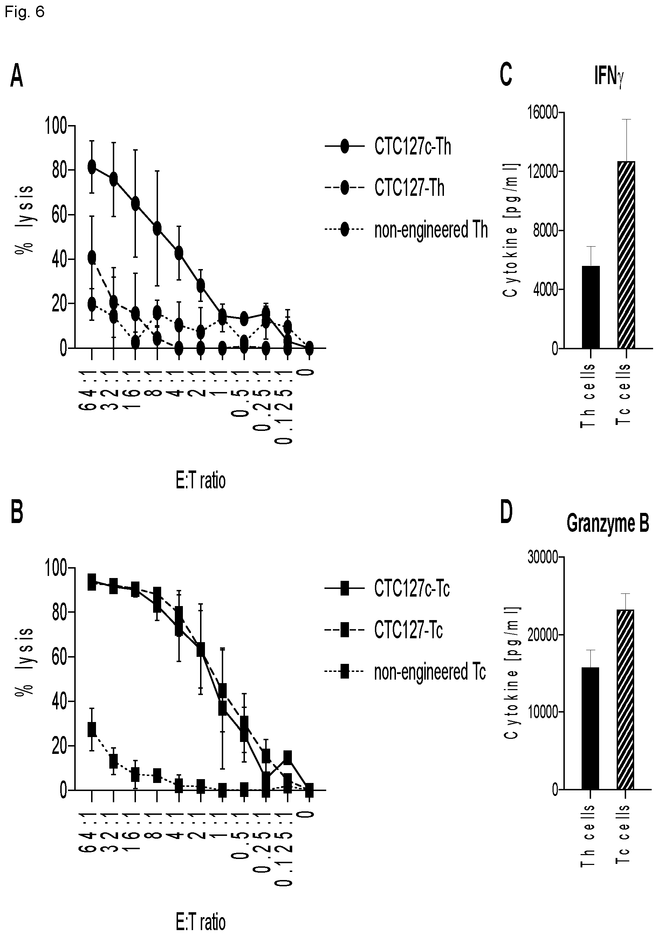

Fig. 6. CTC127c-Th cells are cytolytic, albeit to a lesser extent than Tc cells. Th and Tc cells were separately transduced with CTC127 or CTC127c and used in a killing assay with luciferase-expressing K562-A0101-MAGE-A4 (A & B) or placed in a co-culture with A375 cells (C, D). For the killing assay, a fixed number of target K562-A0101-MAGE-A4 were cultured with titrating numbers of (A) Th or (B) Tc cells. The effector-to-target (E:T) ratio ranged from 64:1 to 0.125:1. After 24h, the luciferase activity of living target cells was quantified and the lysis frequency was calculated by the following formula: 100 - [(signal with T cells) / (signal without T cells) * 100]. C, D) cytokines were measured after 24h of co-culture with A375 cancer cells at a 1:1 ratio. Pooled mean ± SEM of replicates of two T cell donors.

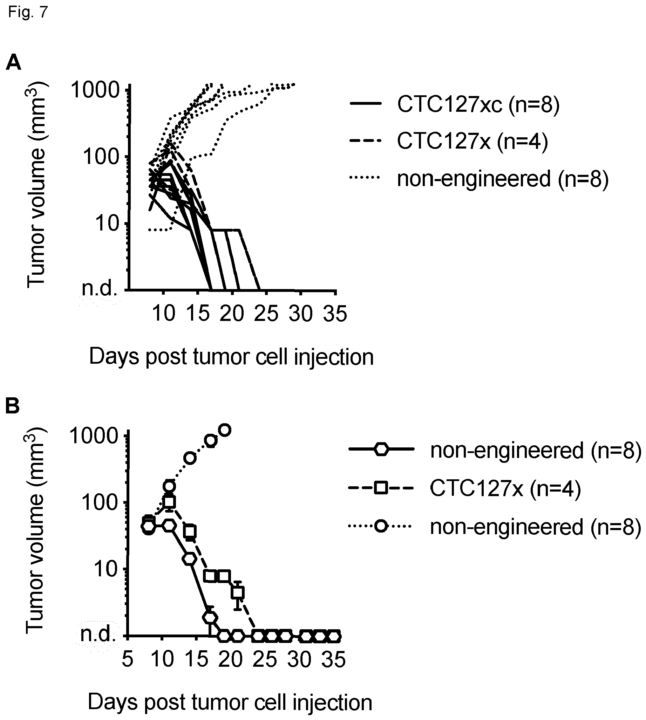

Figure 7. CTC127xc-T cells induced early onset of response and complete rejection of A375 tumors. 5x10⁶ A375 cancer cells were injected s.c. into NSG mice. After 8 days, when tumors had reached an average size of 50 mm³, a mixture of 1x10⁷ engineered Th and Tc cells (expressing CTC127xc or CTC127x) or non-engineered Th and Tc cells (no TCR) were transferred i.v. Tumor growth was measured for 35 days with a caliper. Mice were euthanized after reaching a humane endpoint (tumor > 1500 mm³) A) Tumor growth curves for individual mice. B) Average tumor growth curves for each group (mean ± SEM). Data was pooled from two independent experiments. N.d., not detectable.

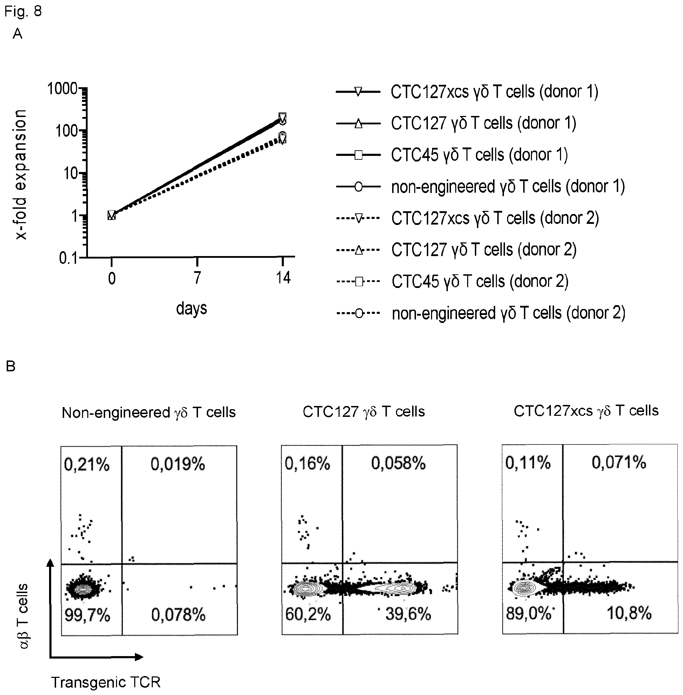

Fig. 8 TCR-engineered gamma delta T cells can be rapidly expanded and highly pure gamma delta T cells harvested at day 14. A Gamma delta T cells were stably engineered with different TCR constructs and/or co-receptor constructs. Engineering and expansion in two different donors are shown. B. After harvest at day 14, alpha beta T cell contamination was below 0.25%. The therapeutic TCR constructs and Co-receptors were strongly expressed in the gamma delta T cells.

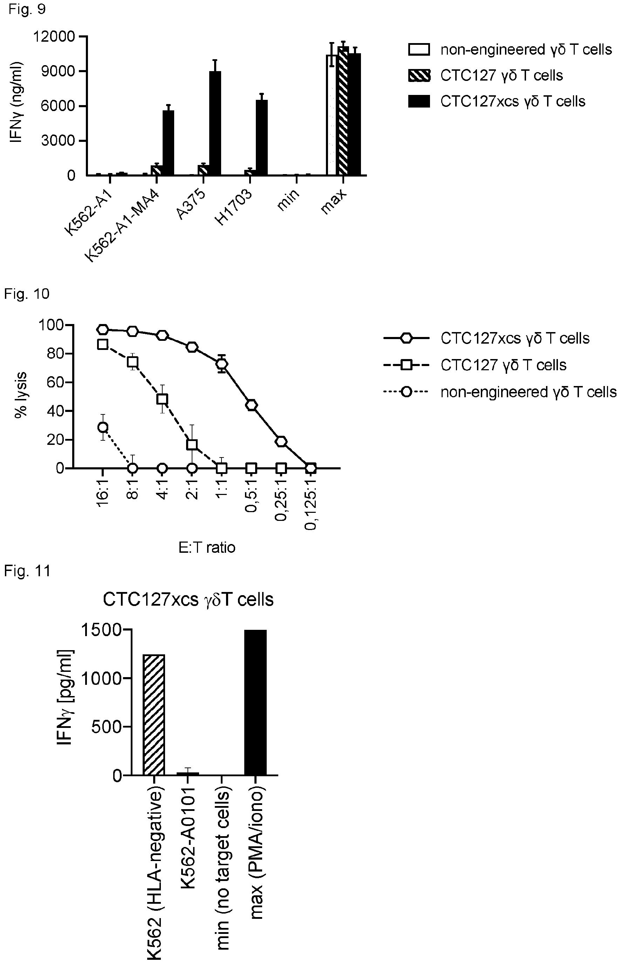

Fig. 9 CD8 co-receptor engineered alpha beta TCR T cells strongly recognize different tumor cell lines upon co-culture. Gamma delta T cells are either non-engineered or engineered to express the TCR CTC127 or they are CD8 co-receptor-engineered to express CTC127xcs. The K562 tumor cell line is engineered to express either HLA-A0101 (K562-A1) or HLA-A01*01 plus the MAGE-A4 antigen (K562-A1-MA4). A375 and H1703 tumor cell lines are naturally positive for HLA-A01*01 and MAGE-A4. Shown are pooled data with gamma delta T cells from two different donors.
Fig. 10 Gamma delta T cells expressing an MHC-I-restricted TCR and a CD8 coreceptor are cytotoxic. Gamma delta T cells were separately transduced with CTC127 or CTC127xcs and used in a killing assay with luciferase-expressing K562-A0101-MAGE-A4. For the killing assay, a fixed number of target K562-A0101-MAGE-A4 were cultured with titrating numbers of gamma delta T cells. The effector-to-target (E:T) ratio ranged from 16:1 to 0.125:1. After 24h, the luciferase activity of living target cells was quantified and the lysis frequency was calculated by the following formula: 100 - [(signal with T cells) / (signal without T cells) * 100]. Pooled mean ± SEM of replicates of two T cell donors.
Fig. 11 Gamma delta T cells can sense HLA loss. Co-culture of CTC127xcs gamma delta T cells with HLA-negative and HLA-positive K562 cell lines shows that IFN-gamma release is induced by HLA-negative cells. IFN-gamma release in the supernatant is measured by ELISA. Shown are representative data from 1 of 2 donors.

### Sequences

| | | |
|---|---|---|
| SEQ ID NO: 1 | amino acid sequence of human CD8 beta chain isoform 5 | |
| SEQ ID NO: 2 | amino acid sequence of human CD8 alpha chain isoform 1 | |
| SEQ ID NO: 3 | T2A | EGRGSLLTCGDVEENPGP |
| SEQ ID NO: 4 | P2A | ATNFSLLKQAGDVEENPGP |
| SEQ ID NO: 5 | E2A | QCTNYALLKLAGDVESNPGP |
| SEQ ID NO: 6 | F2A | VKQTLNFDLLKLAGDVESNPGP |
| SEQ ID NO: 7 | core sequence motif of 2A elements peptides DXEXNPGP | |
| SEQ ID NO: 8 | consensus TEV cleavage site ENLYFQS | |
| SEQ ID NO: 9 | preferred IRES | |
| SEQ ID NO: 10 | nucleic acid segments encoding the CD8 beta chain linked, via a nucleic acid segment encoding a P2A cleavage site, to the nucleic acid segment encoding the CD8 alpha chain, codon-optimized. | |
| SEQ ID NO: 11 | nucleic acid of the invention (operon with CTC127 in the configuration TCRβ-P2A-TCRα-P2A-CD8β-P2A-CD8α) | |
| SEQ ID NO: 12 | comparative nucleic acid in the configuration TCRβ-P2A-TCRα-P2A-CD8α-P2A-CD8β (TCR127) | |
| SEQ ID NO: 13 | comparative nucleic acid in the configuration TCRβ-P2A-TCRα-P2A-CD8α (TCR127) | |
| SEQ ID NO: 14 | nucleic acid in the configuration CD8β-P2A-CD8α--P2A- TCRβ-P2A-TCRα (TCR127) | |
| SEQ ID NO: 15: | | IRES |
| SEQ ID NO: 16 | | amino acid sequence of human CD8 beta chain isoform 1 |
| SEQ ID NO: 17 | | amino acid sequence of human CD8 beta chain isoform 2 |
| SEQ ID NO: 18 | | amino acid sequence of human CD8 beta chain isoform 3 |
| SEQ ID NO: 19 | | amino acid sequence of human CD8 beta chain isoform 4 |
| SEQ ID NO: 20 | | amino acid sequence of human CD8 beta chain isoform 6 |
| SEQ ID NO: 21 | | amino acid sequence of human CD8 beta chain isoform 7 |
| SEQ ID NO: 22 | | amino acid sequence of human CD8 beta chain isoform 8 |
| SEQ ID NO: 23 | | amino acid sequence of human CD8 alpha chain isoform 2 |
| SEQ ID NO: 24 | | amino acid sequence of human CD8 alpha chain isoform 3 |

### Examples

### Example 1 - CD8 co-receptor constructs

### MATERIALS AND METHODS

For the generation of transduced T cells, PBMCs were isolated from fresh blood via gradient centrifugation and T cells contained therein (mixed CD8 and CD4 populations or individual CD8 and/or CD4 populations isolated via magnetic bead sorting) were stimulated with bead-immobilized anti-CD3 and anti-CD28 antibodies and cultivated in RPMI medium supplemented with 10 % FBS, 1 % penicillin/streptomycin, 1 mM sodium pyruvate, 0.1 mM MEM non-essential amino acids and 400 IU/mL human recombinant IL-2. On days 2 and 3 after stimulation, cells were transduced with a retrovirus containing a transgene (SEQ ID NO 11-14). The retrovirus was generated by transfection of GALV cells with plasmid DNA of the transgene(s) using Lipofectamine transfection kit (Invitrogen) according to the manufacturer's instructions . Transduced T cells were further cultivated for 9 to 12 days and used for assays. The expression of the transgenic TCR and coreceptor was assessed by flow cytometry with antibodies against the murine constant TCR beta chain and human CD8a, respectively.

Transduced gamma delta T cells were generated from PBMCs via cultivation with 1µg/ml zoledronate. On days 4 and 5 after stimulation, cells were transduced with a retrovirus containing a transgene (SEQ ID NO 11-14). The retrovirus was generated by transfection of GALV cells with plasmid DNA of the transgene(s). Transduced gamma delta T cells were further cultivated for 14 days and used for assays. The expression of the transgenic TCR and coreceptor was assessed by flow cytometry with antibodies against the murine constant TCR beta chain and human CD8a, respectively.

### RESULTS

### Generation and functional testing of candidate CD8 co-receptor constructs

CTC127 recognizes an epitope of MAGE-A4 presented by HLA-A*01:01. Here, we show that CD8⁺ Tc cells expressing CTC127 efficiently recognized peptide-loaded K562-A0101 target cells in a peptide titration assay and secreted IFNy, a cytokine that correlates with T cell activation and function. Conversely, CD4⁺ Th cells expressing CTC127 failed to recognize the peptide-loaded target cells (Fig. 1) and did not secrete IFNy. This is due to the lack of a CD8 co-receptor. Delivery of a CD8 co-receptor molecule together with CTC127 to Th cells could render them functional against this HLA class I-restricted antigen.

The CD8 co-receptor consists of two subunits (CD8a and CD8b), which are most commonly present as a CD8ab heterodimer on the surface of cytotoxic T cells. Some cells express a CD8a homodimer; however, this is rare. We designed three candidate constructs using the wild-type sequences of the subunits: CD8a alone (as used by Adaptimmune), CD8a followed by CD8b linked via a P2A element (as suggested by Hans Stauss and colleagues ¹⁷ and used by Immatics) and CD8b followed by CD8a linked by a P2A element (constructed by the inventors). In order to test the functionality of the constructs, we co-transduced CTC127 and each of the candidates into isolated CD4⁺ Th or CD8⁺ Tc cells and performed a peptide titration assay to test for functionality of the two T cell subtypes. We observed that all three CD8 co-receptor constructs conferred functionality to Th cells, allowing them to secrete IFNy in response to K562-A0101 loaded with the cognate peptide of CTC127. But the heterodimeric constructs (CD8ab and CD8ba) were superior to the CD8a homodimeric construct with a higher functional avidity (EC₅₀) (Fig. 2A). The EC₅₀ of CD8ab and CD8ba was equivalent. Importantly, the addition of the different CD8 co-receptor constructs to Tc cells did not change the peptide sensitivity (EC₅₀) (Fig. 2B). Thus, delivering TCR and co-receptoron a single construct to all T cells simultaneously without the need to separate them into Th and Tc cells allows an efficient single engineering manufacturing process.

Next, we tested whether Th cells expressing CTC127 and a CD8 co-receptor could recognize MAGE-A4⁺ tumor cells. For this experiment we chose the cancer cell lines A375 and H1703, which naturally express MAGE-A4 and HLA-A*01:01. We also included engineered K562 expressing HLA-A*01:01 and K562 stably expressing the full-length MAGE-A4 gene in addition to A*01:01. We observed that Th cells expressing the homodimeric CD8a construct were not able to recognize the cancer cell lines A375 and H1703, but did recognize K562-A0101-MAGE-A4. These data suggest that the CD8a homodimer can only confer functionality to Th cells when MAGE-A4 is highly expressed by the tumor cells, which was only achieved in the engineered cell line and not in the naturally MAGE-A4-expressing cancer cell lines. Interestingly, the inability of CD8a homodimers to confer functionality in the absence of CD8b has been described in literature ¹⁸ . Both heterodimeric CD8 constructs allowed Th cells to recognize all three cell lines, with the CD8ba construct leading to the highest IFNy secretion (Fig. 3A). The lower recognition of H1703 compared to A375 can be explained by differences in MAGE-A4 expression levels (1626.7 rpkm and 4268.5 rpkm, respectively). Importantly, the addition of the CD8 co-receptor did not lead to non-specific recognition of antigen-negative K562-A0101 cells. We also performed the same experiment using Tc cells and observed that recognition of all cell lines was unaffected by the addition of CD8 co-receptors, supporting our previous peptide titration data that overexpression of CD8 in Tc does not impact antigen recognition (Fig. 3Fig. 3B).

Based on these findings, we selected the CD8ba construct as co-receptor, and all further experiments were performed with the TCR CTC127 and CD8ba co-receptor construct linked together via P2A elements on the same transgene cassette and subsequently termed **CTC127c.**

### Functional profiling of helper T cells expressing CTC127c

Having observed that CTC127c Th cells can specifically recognize MAGE-A4 via the class I allele HLA-A*01:01, we set out to perform a deeper functional profiling of these engineered T cells. Th cells have distinct functions compared to Tc cells. They are generally referred to as "helper" T cells because they activate dendritic cells, macrophages and B cells via cytokines and CD154-CD40 interactions and support the function of Tc cells by secreting cytokines such as IL-2. Furthermore, a subset of Th cells is also capable of cytolysis. By performing a functional profiling, we aimed to determine whether Th cells expressing CTC127c maintained typical helper T cell functions when activated via an HLA class I antigen with the involvement of the added CD8 co-receptor.

We first assessed cytokine secretion in a co-culture with MAGE-A4⁺ A375 cancer cells (to activate the T cells via CTC127c) or anti-CD3/anti-CD28 beads as a positive control (to activate the T cells independently of TCR and co-receptor). With this setup we can observe whether the cytokine profile of CTC127c Th cells differs depending on the activation route. After 24 hours, we measured six cytokines in the supernatants with a multiplex cytometric bead array (CBA). We chose typical cytokines associated with helper and cytotoxic T cell function (IL-2, IL-4, IL-5, IL-13, IFNy, and TNFa). IL-2 is a potent stimulator of Th and Tc cells and supports their survival *in vivo.* IFNγ and TNFα are mediators of cytotoxicity that promote antigen presentation in tumor cells via induction of proteasome activity and MHC expression, which in turn facilitates their detection by T cells. They also activate innate immune cells and have direct tumoricidal effects. IL-4, IL-5, and IL-13 are classical helper type 2 cytokines that have pleiotropic immune functions, such as activation of B cells, dendritic cells and other innate cell types that may boost the anti-tumor function induced by the Tc cells.

After co-culture with A375, only Th cells expressing CTC127c became activated and secreted cytokines beyond basal levels (Fig. 4). Comparing CTC127c-Th cells activated via A375 with the TCR-agnostic positive control, we observed that the levels of most cytokines were similar, suggesting that the presence of the CD8 co-receptor did not significantly alter the qualitative profile of the helper T cells.

Next, we assessed the surface expression of CD154 (CD40L) by Th and Tc cells. CD154 is the major surface molecule expressed by mature Th cells and is essential for activation of B cells (germinal center formation, antibody class-switching and affinity maturation) and dendritic cells (maturation and antigen presentation to Tc cells). In the context of anti-tumor immunity, CD154 plays a role in intra-tumoral dendritic cell and macrophage activation and can promote boosting of T cell responses in the lymph nodes. We measured expression of CD154 after overnight co-culture with A375 cancer cells. As expected, non-engineered Th and Tc cells and CTC127-Th cells showed only a low expression of CD154, which can be interpreted as a basal level. In contrast, 55% of CTC127c-Th cells expressed CD154 in response to A375 cancer cells, indicating antigen-specific activation of the helper T cells. 20% of CTC127- and CTC127c-Tc cells expressed CD154 (Fig. 5). The large disparity in CD154 expression between Th and Tc CTC127c-T cells exemplifies the different functionality of these T cell subsets.

Lastly, we measured the cytolytic capacity of both T cell subsets in order to determine whether CTC127c-Th cells were also able to perform this function, which is normally more prominent in Tc cells. For the killing assay, we used a fixed number of K562 cells expressing HLA-A*01:01, MAGEA4 and luciferase and added T cells in different effector-to-target (E:T) ratios. After 24 hours, we measured the luciferase activity of the remaining live K562 cells and calculated the degree of lysis by the T cells. We observed, as expected, that CTC127-Th cells did not kill cancer cells above background (as background, we considered non-engineered T cells that do not express an engineered TCR, thus designated "non-engineered"). CTC127c-Th cells, however, showed a specific killing capacity persisting until an E:T of 2:1 (Fig. 6A). In contrast, both CTC127- and CTC127c-Tc cells were very efficient at killing the target cancer cells until an E:T ratio of about 0.5:1 (Fig. 6B). The difference in cytolytic capacity between CTC127c-Tc and -Th cells can likely be explained by the higher capacity of Tc cells to secrete IFNy and granzyme B (Fig. 6C, D). IFNy correlates with expression of other cytolytic proteins and can itself trigger apoptosis in cancer cells, and granzyme B is the major protease involved in cytolysis.

Using this suite of functional assays, we characterized CTC127c-Th cells and could observe that they maintain typical "helper" functions of cytokine secretion and CD154 expression and also acquired direct cytolytic capacity, albeit to a lesser extent than CD8⁺ T cells. Usage of both T cell populations in the therapeutic product can leverage the unique capabilities of each subset and provide a beneficial synergistic effect.

### In vivo assessment of CTC127c

Our next step was to evaluate the efficacy of CTC127c-T cells *in vivo* against A375 tumors. Our previous *in vivo* experiments showed that CTC127-Tc cells were able to provide a significant survival benefit to the mice. The following experiments are performed with a variant of CTC127, CTC127x, which led to complete responses *in vivo.* In order to assess the effect of CD8 co-receptor expression on tumor rejection, we compared CTC127x with CTC127xc (TCR without and with coreceptor).

We inoculated NSG mice subcutaneously with A375 tumors and waited 8 days for tumors to establish at an average size of 50 mm³ before transferring intravenously a mixed population of Tc and Th cells (each mixture in three groups: CTC127xc, CTC127x and no TCR). We then followed tumor growth in the mice for a total of 35 days. Mice that received non-engineered T cells (no TCR) did not control the A375 tumors and all reached a humane endpoint at about 20 days post tumor cell injection. In contrast, both groups that received CTC127-engineered T cells completely rejected the tumors and remained tumor-free until the end of the observation period (Fig. 7). The tumor rejection was 33 % faster in the CTC127xc group compared to the CTC127x group (10 days and 15 days, respectively), showing a significant benefit of employing the co-receptor, which allows for synergistic Tc and Th functions *in vivo.* The mice in the CTC127xc group remained tumor-free until the end of the experiment (35 days).

These experiments showed that the use of the co-receptor in CTC127xc allowed for the synergistic function of Tc and Th cells, resulting in a faster complete tumor clearance in 100% of the mice.

### CONCLUSION

In this report, we showed the development of a toolbox technology that has the potential to greatly improve clinical efficacy of TCR-engineered T cells.

We built a CD8 co-receptor construct containing the beta and alpha subunits in reversed order and benchmarked it against two other constructs: 1) CD8a homodimer as used by Adaptimmune and 2) CD8ab heterodimer as used by Immatics. Our CD8ba construct showed superior functional avidity compared to the Adaptimmune CD8a construct and better tumor cell recognition than the Immatics CD8ab construct.

The CD8ba co-receptor conferred full functionality to Th cells expressing HLA class I-restricted TCRs with no deleterious effect on Tc cells. We showed that CTC127c-Th cells exerted full helper function in response to MAGE-A4⁺ cancer cells, including cytotoxic and helper cytokine secretion and CD154 expression. Further, they were able to directly kill cancer cells. *In vivo,* CTC127xc T cells led to complete and durable rejection of MAGE-A4⁺ A375 tumors, which was 33 % faster than CTC127x T cells (without the co-receptor).

The data in this report were mostly generated in conjunction with CTC127. However, in general, the CD8ba co-receptor technology is agnostic to the TCR and can be employed with any other TCR. Importantly, TCR and co-receptor can be placed in the same construct and can be delivered simultaneously to the T cells, eliminating the need for additional engineering steps during manufacturing and eliminating the generation of T cells expressing only one of the constructs. Furthermore, TCR and CD8ba co-receptor can be employed in cell types such as γδT cells or NK cells, which don't express TCR co-receptors, and provide them with anti-tumor functionality. This is particularly useful for allogeneic approaches utilizing γδT or NK cells engineered with class I-restricted TCRs.

The CD8ba co-receptor technology we developed allows us to take advantage of the full potential of TCR-T cell therapies by employing both Tc and Th cell subsets with distinct and synergistic functions that are essential for success against solid tumors. Additionally, it opens up the possibility for the use of δγT and NK cells in allogeneic approaches.

### Example 2: Gamma delta T cells

Gamma delta T cells were generated from healthy donor PBMCs by activation with 1 µg/mL zoledronate and transduced with retrovirus containing TCRs and/or the coreceptor constructs described herein. The retrovirus was generated by transfection of GALV cells with plasmid DNA of the transgene(s) using Lipofectamine transfection kit (Invitrogen) according to the manufacturer's instructions. The T cells were cultured for 14 to 28 days in medium containing recombinant human IL-7 and IL-15 (feeding every 2 - 3 days) prior to use in experiments. Gamma delta T cells were able to expand about 100-fold in the 14 days of culture regardless of which engineering construct was used (Fig. 8). Importantly, the contamination by alpha beta T cells was below 0.25 % in the final cell population (Fig. 8). Wildtype gamma delta T cells do not express the CD8 coreceptor, thus, they are not fully functional if only engineered with an MHC class I restricted TCR, such as CTC127. Only when co-engineered with a CD8 coreceptor (CTC127xcs), the gamma delta T cells were able to recognize cancer cells expressing MAGE-A4 via HLA-A*01:01 (Fig. 9). In these experiments, gamma delta T cells and tumor cells were co-cultured for 24 hours, after which IFNγ was measured in the supernatant via ELISA as a surrogate for tumor cell recognition and gamma delta T cell activation. In order to access the cytotoxic capacity of gamma delta T cells expressing the CTC127 and coreceptor constructs, a fixed number of K562 cells expressing HLA-A*01:01, MAGE-A4 and luciferase were cultivated with gamma delta T cells in different effector-to-target (E:T) ratios. After 24 hours, the luciferase activity of the remaining live K562 cells was measured and the degree of lysis by the gamma delta T cells was calculated. CTC127xcs gamma delta T cells showed a superior cytolytic capacity when compared to CTC127 and non-engineered gamma delta T cells (Fig. 10). Thus, the addition of the CD8 coreceptor is essential in order to unlock the full potential of TCR-engineered gamma delta T cells against cancer. Gamma delta T cells can additionally sense HLA loss in target cells and this feature may provide an additional mode of action if tumor cells try to escape through downregulation of HLA. This is exemplified by the recognition of the HLA-negative cell line K562, but not of its HLA-transduced counterpart (K562-A0101) after a 24-hour coculture (Fig. 11).

### References

1. S. Ottaviani, D. Colau, P. van der Bruggen, P. van der Bruggen, Cancer Immunology, Immunotherapy. 55, 867-872 (2006).
2. T. Kobayashi et al., Tissue Antigens. 62, 426-432 (2003).
3. M. Ishihara et al., BMC Cancer. 20, 606 (2020).
4. K. lura et al., Virchows Arch. 471, 383-392 (2017).
5. C. J. Turtle et al., J Clin Invest. 126, 2123-38 (2016).
6. C. J. Turtle et al., Sci Transl Med. 8, 355ra116 (2016).
7. D. Sommermeyer et al., Leukemia. 30, 492-500 (2016).
8. J. R. Veatch et al., J Clin Invest. 128, 1563-1568 (2018).
9. L. Wooldridge et al., J Biol Chem. 280, 27491-501 (2005).
10. B. Laugel et al., J Biol Chem. 282, 23799-810 (2007).
11. D. K. Cole et al., Immunology. 137, 139-48 (2012).
12. R. Campanelli et al., Int Immunol. 14, 39-44 (2002).
13. WO 2020/049496 A1.
14. WO 2019/204662 A1.
15. WO 2020/208346 A1.
16. X. X. Wang et al., Proceedings of the National Academy of Sciences. 108, 15960-15965 (2011).
17. M. L. L. Donnelly et al., J Gen Virol. 82, 1027-1041 (2001).
18. C. U. Hellen, P. Sarnow, Genes Dev. 15, 1593-612 (2001).
19. D. Sommermeyer, W. Uckert, Journal of immunology. 184, 6223-31 (2010).
20. C. J. Cohen, Y. Zhao, Z. Zheng, S. A. Rosenberg, R. A. Morgan, Cancer Res. 66, 8878-86 (2006).
21. C. J. Cohen et al., Cancer Res (2007), doi:10.1158/0008-5472.CAN-06-3986.
22. J. Kuball et al., Blood. 109, 2331-8 (2007).
23. K. B. J. Scholten et al., Clin Immunol. 119, 135-45 (2006).
24. M. Leisegang et al., J Mol Med (Berl). 86, 573-83 (2008).
25. WO 2017/158019 A1.
26. WO 2009/003671 A2.
27. WO 2017/158029 A1.
28. K. P. Nishimoto et al., Clin Transl Immunology. 11 (2022), doi:10.1002/cti2.1373.
29. W. K. Tan, J. Tay, J. Zeng, M. Zheng, S. Wang, "Minireview Open Access Expansion of Gamma Delta T Cells-A Short Review on Bisphospho-nate and K562-Based Methods Gamma Delta T cells As the Next Generation of Effector Cells for Immunotherapy" (2018).
30. WO 2022/243514 A1.
31. Z. Sebestyén et al., J Immunol. 180, 7736-46 (2008).
32. C. Govers et al., The Journal of Immunology. 193, 5315-5326 (2014).
33. US 2003219463 A1
34. L.T. van der Veeken et al, Cancer Res. 66(6), 3331-3337 (2006),
35. L.T. van der Veeken et al, J Immunol. 182(1), 164-170 (2009)
36. WO2019/104269 A1

## Claims

1. A nucleic acid encoding a CD8 alpha beta co-receptor, wherein alpha chain and beta chain are not covalently linked, wherein the nucleic acid comprises an operon capable of mediating expression of the CD8 alpha and CD8 beta chain under the control of one promotor from a polycistronic mRNA,
wherein the nucleic acid segment encoding the CD8 beta chain is more proximal to the promotor than the nucleic acid segment encoding the CD8 alpha chain.

2. The nucleic acid of claim 1, wherein the nucleic acid segment encoding the CD8 beta chain is linked to the nucleic acid segment encoding the CD8 alpha chain via a nucleic acid segment encoding a cleavage site.

3. The nucleic acid of claim 2,wherein the cleavage site is a self-cleaving peptide selected from the group comprising a P2A element, a T2A element, an E2A element and an F2A element,
preferably, a P2A element.

4. The nucleic acid of claim 1, wherein an internal ribosome entry site (IRES) is located between the nucleic acid segment encoding the CD8 beta chain and the nucleic acid segment encoding the CD8 alpha chain.

5. The nucleic acid of any of claims 1-4, further encoding a TCR alpha chain construct and a TCR beta chain construct or a chimeric antigen receptor, preferably, a TCR alpha chain construct and a TCR beta chain construct.

6. The nucleic acid of claim 5, wherein the operon comprises nucleic acid segments encoding
• the CD8 alpha chain and the CD8 beta chain and
• a TCR alpha chain construct and a TCR beta chain construct or a chimeric antigen receptor, preferably, a TCR alpha chain construct and a TCR beta chain construct.

7. The nucleic acid of any of claims 1-6, wherein the operon is flanked by inverted repeats capable of being mobilized by a transposase, preferably, by a Sleeping Beauty transposase such as SB100X.

8. The nucleic acid of any of claims 1-7 that is a minicircle, a plasmid, doggybone DNA or RNA, preferably, a minicircle.

9. A cell comprising the nucleic acid of any of claims 1-8.

10. The cell of claim 9, wherein the cell further expresses a class-I-restricted alpha beta T cell receptor construct, wherein the cell is selected from the group consisting of an immune cell such as a CD4 T cell and/or CD8 T cell, gamma delta T cell, NK cell, NKT cell, MAIT, ILC, monocyte, macrophage and B-cell,
preferably, a CD4 T cell or a gamma delta T cell.

11. An immune cell expressing
a) a TCR alpha chain construct and a TCR beta chain construct of a MHC class I restricted TCR construct and
b) a CD8 co-receptor comprising both CD8 alpha and CD8 beta chain,
wherein the immune cell is selected from the group consisting of a gamma delta T cell, an NK cell, an NKT cell, a mucosa-associated invariant T (MAIT) cell, an innate lymphoid cell (ILC), a CD4+ T helper cell, a monocyte, a macrophage and a B cell,
wherein the cell optionally comprises the nucleic acid of any of claims 1-8.

12. The immune cell of claim 11 that is a gamma delta T cell.

13. The immune cell of claim 11 that is a CD4+ T helper cell.

14. A pharmaceutical composition comprising the nucleic acid of any of claims 1-8 or the cell of any of claims 9-13, and optionally, a suitable buffer and/or excipient.

15. The pharmaceutical composition of claim 14 for use in treatment of cancer or an infectious disease, preferably, in adoptive T cell therapy of cancer,
wherein, optionally, the cells for adoptive T cell therapy are allogeneic gamma delta T cells of claim 12.
